# EUROPEAN PATENT SPECIFICATION

(11) **EP 1 340 085 B1**
(45) Date of publication and mention of the grant of the patent: **30.07.2008**
(21) Application number: 01976048.7
(22) Date of filing: 12.10.2001
(51) Int. Cl.: G01N 33/569

(54) **ASSAY FOR DIRECTLY DETECTING A BIOLOGICAL CELL IN A BODY FLUID SAMPLE**
TEST ZUM DIREKTEN NACHWEISEN EINER BIOLOGISCHEN ZELLE IN EINER KÖRPERFLÜSSIGKEITSPROBE
ANALYSE PERMETTANT DE DETECTER DIRECTEMENT UNE CELLULE BIOLOGIQUE DANS UN ECHANTILLON DE FLUIDE CORPOREL

(30) Priority: 17.10.2000 DK 200001546; 23.10.2000 US 242135 P
(43) Date of publication of application: 03.09.2003
(73) Proprietor: Besst-Test APS, 8000 Aarhus C (DK)
(72) Inventor: LASSEN, Michael, Rud, DK-2960 Rungsted Kyst (DK); BREINDAHL, Morten, DK-2800 Kgs. Lyngby (DK)
(74) Representative: HOEIBERG A/S
(86) International application number: PCT/DK2001/000673
(87) International publication number: WO 2002/033418

(56) References cited:
- EP-A1- 0 267 317
- EP-A2- 0 577 092
- WO-A1-90/15327
- WO-A1-97/13150
- US-A- 5 587 294

## Description

### Technical Field

The present invention relates to a method for rapid detection of a biological cell and/or a biological particle contained in a fluid sample. The method may be used for rapidly diagnosing a condition in an individual resulting from an infection by a virus, a fungus or a bacteria.

The method comprises the further steps of detecting a plurality of infection and/or inflammatory response agents, preferably cytokines, and performing a profile of such agents. The profile is a further indication of the condition being diagnosed. The method for detecting a plurality of infection response agents, preferably cytokines, including the step of performing a profile of such agents, may also be carried out independently of the method for rapidly diagnosing a condition in an individual resulting from an infection by an infectious agent including a virus, a fungus and a bacteria.

The method further comprises a further step of detecting at least one inflammatory response agent, such as at least one immune system agent, preferably autoantibodies, and optionally performing a profile of such agents. The agent may be the only or the first indication of the condition being diagnosed or it may contribute as a further indication of the condition being diagnosed.

The methods of the invention are performed by contacting i) a body fluid sample potentially comprising an infectious agent and/or an infection and/or inflammatory response agent, including cytokines, with ii) a targeting species that is preferably quantifiably detectable and capable of specifically recognising a predetermined infectious agent and/or a predetermined infection response agent. It is preferred that the contacting takes place essentially without pretreatment of the body fluid sample.

The targeting species preferably comprises an antibody capable of contacting one or both of an infectious agent and an infection response agent. The targeting species may also comprise a visible label capable of being detected when the complex of the targeting species with either one or both of an infectious agent and an infection response agents is in contact with a solid test area on e.g. a lateral flow device.

### Background of the Invention

Antigens from microbial cells have been detected in the prior art. United States Patent 4,663,277 relates to a method for detecting a virus by means of an immunoassay in which an extended solid phase coated with antiviral antibody is employed to bind and remove virions from a specimen by forming an immuno-complex with antigens of said virions, a mobile solid phase comprising a dispersion of microspheres coated with the antiviral antibody is used to bind said microspheres to antigens associated with said immuno-complex, and the presence of bound microspheres is detected. The detection sensitivity is amplified by using microspheres comprising a dye or a label. The extended solid phase may be in the form of a dipstick, syringe, tube or container that can be easily contacted with the specimen. A virus detection kit provides the extended solid phase and mobile solid phases, each coated with antiviral antibodies.

In one embodiment the invention disclosed in US 4,663,277 pertains to a method for detection of viruses in a specimen and comprises the steps of i) treating the specimen to remove undesired components ii) contacting the specimen with a solid phase support having conjugated thereto antiviral antibody capable of forming immuno-complexes with antigens characteristic of the viruses to be detected, iii) separating the solid phase support from the specimen, iv) contacting the separated solid phase support with a mobile solid phase consisting of dispersed microspheres smaller than 0.1 µm and labelled with metal elements and having conjugated thereto the antiviral antibody that enables the binding of said microspheres to said immuno-complexes, v) separating the unbound mobile solid phase from the solid phase support; and vi) measuring the presence of microspheres bound to said solid phase support by X-ray fluorescence, thereby detecting or determining the presence of viruses in said specimen.

An antispecies antibody is covalently bound to the solid phase support as well as to the mobile solid phase, and an antiviral antibody that forms an immuno-complex with the antispecies antibody is coupled therewith, whereby an antiviral antibody capable of forming immuno-complexes with antigens of viruses to be detected is conjugated to said solid phase support and to said mobile solid phase.

In one embodiment of the invention a plurality of different antiviral antibodies capable of forming complexes with corresponding antigens of different types of viruses are conjugated to the solid phase support as well as to the mobile solid phase, whereby the presence of one or more of a plurality of different types of viruses in the specimen can be detected at the same time.

United States Patent 4,740,467 discloses a method for diagnosing syphilis and other treponematoses infections such as yaws and pinta. The method involves admixing i) a biological sample, such as lesion exudate, cerebrospinal fluid, serum, urine, amniotic fluid, synovial fluid or tissue homogenate from a person suspected of having syphilis, yaws or pinta together with ii) a reagent of monoclonal antibodies which are specific for antigens of virulent subspecies of Treponema pallidum, including pertenue, endemicum, carateum and pallidum. If Treponema pallidum, the causative organism of syphilis, is present, an immunological specific binding reaction will occur between the monoclonal antibodies and antigenic sites on T. pallidum cells. A positive immunoreaction is detected directly by a variety of techniques including radioimmunoassay, fluorescent immunoassay, enzyme linked immunosorbent assay, agglutination reactions, and complement consumption tests.

United States Patent 5,290,677 discloses a method for detecting hepatitis A virus by capturing whole virus particles with antibodies specific to hepatitis A virus. In subsequent steps the method comprises generating a cDNA copy of the RNA by reverse transcription in the presence of a primer having a predetermined sequence, amplifying the cDNA by a polymerase chain reaction, and detecting the amplified cDNA by hybridization with probes of a predetermined sequence, or by detection of label bound to the primer, wherein the presence of detectable hybridization or amplification indicates the presence of hepatitis A virus. It is disclosed that samples which contain free virus (for example, stool, environmental samples, or other fomite associated material) may be selectively removed from adventitious material by immunoselection of whole virus using a high titer anti-HAV antibody coated onto a solid phase. The viral RNA is then denatured in the presence of a specific primers, and the viral RNA is reverse transcribed to cDNA using standard methodology.

Further examples of diagnostic methods pertaining to the detection of microbial cells are disclosed e.g. in United States Patent 6,077,665 relating to a rapid assay for infection in immunodeficient patients such as neonates or immunocompromised patients (e.g. HIV or transplant patients). The method allows diagnosis at initial evaluation, such that antibiotic treatment and confinement to an intensive care unit can be avoided for uninfected patients. The assay can be used for sepsis diagnosis including the detection of bacterial, viral, or fungal colonization of the blood stream, cerebrospinal fluid (CSF), or urinary tract. The method is based on the measurement of polymorphonuclear leukocyte (PMN, neutrophil) CD11b (Mac-1, CR3) levels by flow cytometry or laser scanning microscopy in whole blood samples.

United States Patent 5,965,354 relates to a method and immunodiagnostic test kits for diagnosing herpes simplex virus infection. The methods and kits employ type-specific or type-common antigens in a single-step assay format. In one embodiment the method of the invention comprises the steps of i) contacting a biological sample from a human suspected of containing antibodies to herpes simplex virus with one or more purified herpes simplex virus polypeptides bound to a solid support, under conditions that allow herpes simplex virus antibodies, when present in the biological sample, to bind to said herpes simplex virus polypeptides, and ii) detecting the presence or absence of bound antibodies as an indication of the presence or absence of herpes simplex virus, wherein said detecting is done by using at least one detectably labeled anti-human immunoglobulin antibody.

United States Patent 5,939,254 discloses specific primers that amplify a portion of the 3'-noncoding regions of a dengue virus, and a method of using these primers in a rapid reverse transcriptase-polymerase chain reaction (RT-PCR) for specific detection of dengue viruses.

United States Patent 5,919,616 relates to serological detection of a herpes simplex virus infection by means of reaction of a patient serum sample potentially containing virus antibody with a virus specific peptide that may be used in an assay including an enzyme linked immunosorbent assay (ELISA).

United States Patent 5,744,299 is concerned with a method for evaluating a biological sample for the presence or absence of human parainfluenza virus and for the quantitation of the virus. The method comprises the steps of isolating RNA from the biological sample, generating cDNA from the isolated RNA, amplifying the generated cDNA, and determining virus infection by detecting the amplified sequence.

United States Patent 5,695,930 relates to a method for detecting antibodies to a human immunodeficiency virus and comprises the steps of i) contacting saliva from a human with p17 protein from human immunodeficiency virus bound to a nitrocellulose-containing solid support for a time and under conditions sufficient for an antibody in the saliva to said antigen to form a complex therewith, and ii) subjecting the complex to detecting means in order to detect the complex.

United States Patent 5,660,979 discloses a method for determining virus replication in human cells by human retrovirus using RNA amplification and comprises the step of detecting the hybridization of an RNA probe which specifically hybridizes with spliced RNA and not with genomic RNA. The method permits early detection of RNA replication resulting from primary infection without detecting non-replicating virus.

United States Patent 5,643,714 relates to HTLV gp21 envelope protein specific peptides for use in diagnostic assays for detecting and confirming HTLV infection in human sera. The invention also pertains to a kit for detecting the presence of HTLV infection in a human serum sample. The kit comprises i) a solid support and, ii) a peptide antigen attached to the solid support in a reaction zone, and iii) reporter means for detecting the presence of human antibodies bound to the support.

United States Patent 5,593,849 discloses an immunochemical assay that uses enzyme-linked immunosorbence to detect the presence of antibodies against environmental protein sequences that mimic the human opioid peptide dynorphin in a human body fluid sample. The assay makes it possible to correlate and diagnose psychobiological or medical disorders related to alterations in the normal levels of dynorphin peptides or their receptors.

United States Patent 5,587,285 describes a highly sensitive anti-HIV antibody detection assay. The assay detects the presence of anti-HIV antibodies through the use of a non-denatured HIV antigenic determinant which immunoreactivity binds anti-HIV antibodies in a biological sample. The non-denatured HIV antigenic determinant has provided a means for detecting anti-HIV antibodies in serum samples testing seronegative for the presence of HIV antibodies directed against denatured HIV antigens.

United States Patent 5,565,319 relates to compositions derived from a viral isolate of feline T-lymphotropic lentivirus (FTLV) and antibodies to antigenic sites on the virus. The compositions are useful in a variety of techniques for the detection of and vaccination against FTLV. Detection methods disclosed include immunoassays. In one embodiment there is provided an enzyme-linked immunosorbent assay (ELISA) for detecting Feline Immunodeficiency Virus (FIV) antibodies. The assay comprises a solid phase coated with FIV antigen, wherein FIV antibodies in a sample exposed to the solid phase will bind to the antigen; and a detectable label conjugate which will bind to FIV antibodies bound to the solid phase.

United States Patent 5,487,969 pertains to a method for detecting the presence of herpes B virus in an individual and comprises the steps of i) obtaining a sample from an individual suspected of being infected with herpes B virus, ii) extracting DNA from any herpes B virus, iii) amplifying segments of the extracted DNA by using predetermined primer sequences, iv) analyzing the amplified DNA segments by means of e.g. digesting the amplified DNA segments with a restriction enzyme, or by hybridizing the amplified DNA segments with a labeled oligonucleotide probe.

United States Patent 5,225,322 relates to a method for detecting an antigen in a test sample suspected of containing said antigen, and simultaneously determining a fingerprint of antibodies specific for said antigen. The method comprises the steps of i) providing polyclonal antibodies specific for the antigen in question, ii) separating the polyclonal antibodies from each other according to the electrical charge of individual antibodies, iii) binding the separated antibodies to a solid support so that the antibodies separated in step ii) maintain the same relative position with respect to each other on the solid support, the relative position of the antibodies forming a fingerprint of antibodies specific for said antigen, iv) contacting the antibodies bound in step iii) with a test sample suspected of containing the antigen under conditions selected to allow binding of the antigen to the antibodies bound in step iii), v) contacting antigen bound in step iv) with detectably labeled antibodies specific for the antigen, under conditions selected to allow binding of said detectably labeled antibodies to said antigen, and vi) detecting the detectably labeled antibody as an indication of the presence of said antigen in the test sample, and revealing the fingerprint of antibodies that are specific for the antigen when the antigen is present in the test sample.

United States Patent 5,212,062 is related to a method for detecting antigens from Chlamydia psittaci or Chlamydia trachomatis in a sample. The method comprises the steps of i) contacting a sample with a predetermined monoclonal antibody affixed to a solid support for a time and under conditions sufficient to form an immune complex on said support, ii) contacting the support with an antibody which binds to said antigen in said immune complex for a time and under conditions sufficient for binding to occur, and iii) detecting the presence of said immune complex as an indication of the presence of Chlamydia psittaci or Chlamydia trachomatis antigen in said sample.

United States Patent 5,155,021 is directed to a method for the determination of a herpes simplex virus and comprises a first step i) of contacting a specimen suspected of containing herpes simplex viral antigen with polymeric particles which have a surface area of from about 0.1 to about 600 m²/g of particles. Each particle is substantially free of any chemical or biological material and has an average diameter of from about 0.01 to about 10 µm. The particles are capable of directly binding herpes simplex viral antigen. Within about 10 minutes of contacting step i) A, herpes simplex viral antigen directly bound to the particles are contacted in a second step ii) with herpes simplex viral antibody so as to form an immunological complex on said particles. The bound complex is then separated from uncomplexed herpes simplex viral antibody by using a microporous membrane having an average pore size of from about 0.1 to about 20 µm, and the complex is determined as an indication of the presence of herpes simplex virus in said specimen. The method is carried out within about 30 minutes.

United States Patent 5,093,230 relates to an assay method for detecting IgM antibodies to a retrovirus selected from the group consisting of HIV-1, HIV-2, HTLV-I, and HTLV-II. The method is carried out within 70 minutes and comprises the steps of i) contacting nitrocellulose paper containing blotted, resolved retrovirus antigen protein obtained from gel electrophorectically resolved viral lysate with a test sample, and incubating under predetermined conditions the nitrocellulose paper and test sample to permit binding of antibodies present in the sample to the protein on the nitrocellulose paper, iii) contacting the incubated nitrocellulose paper of step i) with an anti-IgM enzyme conjugated antiserum reactive with said antibodies, and incubating to permit binding of the antiserum to said antibodies, iii) contacting the incubated nitrocellulose paper of step ii) with an enzyme substrate specific for the enzyme of step ii), and incubating to produce a colour, iv) stopping the colour producing reaction of step iii); and v) evaluating the amount of colour produced as an indication of the presence of antibodies to the viral lysate.

A plurality of cytokines have been detected in the prior art. United States Patent 5,587,294 relates to a method for measuring endogenous cytokines in blood. Cytokines are measured in the presence of substances that bind the cytokines and causes conventional methods to give inaccurate results. The invention also pertains to non-invasive measurement of cytokines in biological fluids such as saliva and nasal secretions. In one embodiment the invention relates to a method for monitoring cytokine therapy in a human, wherein the cytokine is able to bind a carrier molecule, with the proviso that the cytokine is not IL-1. The method comprises the steps of i) obtaining a human body fluid sample potentially comprising a cytokine, ii) forming an assay mixture by combining the sample from step i) with a) an antibody capable of binding specifically to substantially all of the cytokine, wherein the antibody is immobilized on a solid phase support, and b) a labeled binding epitope of the cytokine, wherein the labeled binding epitope competes with the cytokine for antibody binding, iii) incubating the assay mixture to allow the immobilized antibody to bind specifically to either the cytokine or the labeled binding epitope, iv) washing unbound, labeled binding epitope from the solid phase support, v) detecting bound label on the solid phase support, vi) determining the amount of the cytokine in the sample. In a further step the determination of the amount of cytokine is compared to the amount of the cytokine in the sample with a determination of the cytokine in a previous body fluid sample. Preferred cytokines are interleukin-2, interleukin-6, interferon-α, interferon-γ and tumor necrosis factor-α.

### Summary of the invention

In a first aspect there is provided a kit for directly detecting a predetermined biological cell present in a sample in an amount of less than about 2000 per microlitre (10⁻⁶ litre), said kit comprising
i) a solid support, and
ii) a plurality of a first targeting species bound to the solid support, said targeting species being capable of directly detecting said predetemined biological cell when it is present in a sample that is brought into contact with the solid support, and
iii) a conjugate comprising a polymeric carrier molecule bound to at least
   a) one first and/or second targeting species being capable of directly detecting said predetemined biological cell when it is present in a sample that is brought into contact with the solid support, and
   b) at least one labelling species.

In further aspects the invention relates to a method for detecting a biological cell in a sample and a method for diagnosing an infection in an individual.

The method of detecting a predetermined biological cell present in a sample, preferably a body fluid sample, comprises the steps of
i) contacting the sample with the kit according to the invention, and
ii) detecting a targeting species capable of targeting the predetermined biological cell,
wherein the detection of the targeting species is indicative of the presence of the biological cell in the sample.

In another aspect there is provided a method for detecting at least one predetermined inflammatory indicator present In a sample in an amount of less than 100 nanograms (100 x 10⁻⁹ grams) per millilitre (10⁻³ litre). The method comprises the steps of
i) contacting the sample with a kit comprising
   a) a solid support, and
   b) a plurality of a first targeting species bound to the solid support, said targeting species being capable of directly detecting said predetemined biological cell when it is present in a sample that is brought into contact with the solid support, and
   c) a conjugate comprising a polymeric carrier molecule bound to i) at least one first and/or second targeting species capable of directly detecting said predetemined biological cell when it is present in a sample that is brought into contact with the solid support, and ii) at least one labelling species, and
ii) detecting a targeting species capable of targeting the predetermined inflammatory indicator,
   wherein the detection of the targeting species is indicative of the presence of the predetermined inflammatory indicator in the sample.

By the term inflammatory indicator or agent is meant an indicator of inflammatory and/or immune system activity, such indicators for example being cytokines and autoantibodies.

In a further aspect there is provided a method for diagnosing an infectious condition in an individual, said method comprising the steps of
i) detecting a predetermined biological cell present in a body fluid sample according to the method of the invention, and
ii) diagnosing said infectious condition.

In yet a further aspect there is provided a method for diagnosing an inflammatory condition in an individual, said method comprising the steps of
iii) detecting at least one predetermined biological particle present in a body fluid sample according to the method of the invention, and
iv) diagnosing said inflammatory condition.

In a still further aspect the invention pertains to a method for diagnosing an infectious condition in an individual, said method comprising the steps of
i) detecting a predetermined biological cell present in a body fluid sample according to a method of the invention,
ii) detecting a predetermined inflammatory indicator present in a body fluid sample according to a method of the invention, and
iii) diagnosing said infectious condition.

Still further aspects of the invention relates to a kit according to the invention for use in i) a method for detecting a predetermined biological cell or a predetermined inflammatory indicator, ii) a method for diagnosing an Infectious condition in an individual, and/or a method for treating an infectious condition in an individual.

### Detailed Description of the Invention

In one preferred embodiment the present invention relates to a kit for directly detecting a predetermined biological cell present in a sample in an amount of less than about 2000 per microlitre (10⁻⁶ litre), said kit comprising
i) a solid support, and
ii) a plurality of a first targeting species bound to the solid support, said targeting species being capable of directly detecting said predetemined biological cell when it is present in a sample that is brought into contact with the solid support, and
iii) a conjugate comprising a polymeric carrier molecule bound to at least
   a) one first and/or second targeting species being capable of directly detecting said predetemined biological cell when it is present in a sample that is brought into contact with the solid support, and
   b) at least one labelling species,
wherein the polymeric carrier molecule comprises a plurality of at least one reactive, functional group, at least one connecting moiety attached to the at least one reactive, functional group, and at least one molecular species selected from the group of molecular species consisting of targeting species and labelling species,
wherein each of the molecular species comprises at least one functional group that is reactive with at least one connecting moiety attached to the reagent, and
wherein the conjugate comprises at least one molecular species covalently attached thereto via a connecting moiety.

The polymeric carrier molecule preferably comprises reactive, functional groups in an amount of from about 5 to about 5,000 µmoles per gram of polymeric carrier.

In another embodiment the polymeric carrier molecule comprising e.g. a dextran chain according to the invention comprises less than about 400 labelling species, preferably in the form of visibly detectable targeting species or fluorescently detectable labelling species, such as less than 380 labelling species, for example less than 360 labelling species, such as less than 340 labelling species, for example less than 320 labelling species, such as less than 300 labelling species, for example less than 280 labelling species, such as less than 260 labelling species, for example less than 240 labelling species, such as less than 220 labelling species, for example less than 200 labelling species, such as less than 180 labelling species, for example less than 160 labelling species, such as less than 140 labelling species, for example less than 120 labelling species, such as less than 100 labelling species, for example less than 80 labelling species, such as less than 70 labelling species, for example less than 60 labelling species, such as less than 50 labelling species, for example less than 40 labelling species, such as less than 30 labelling species, for example less than 25 labelling species, such as less than 20 labelling species, for example less than 15 labelling species, such as less than 12 labelling species, for example less than 10 labelling species, such as less than 8 labelling species, for example less than 4 labelling species, such as less than 3 labelling species, for example less than 2 labelling species.

The molecular weight of a polymeric dextran chain is preferably about 500,000 Da, but molecular weights from about 100,000 Da to about 900.000 Da can also be used.

Each dextran chain in one embodiment comprises approximately 2,700 glucose units of which 20-22% are activated, preferably with divinyl sulfon, although other connecting moieties can also be used as described herein below in detail.

In one embodiment about half of the about 600 connecting moieties, preferably, but not limited to, divinyl sulfon groups, per dextran chain react with targeting species and labelling species according to the invention, and this provides the figure of less than about 400 labelling species per dextran chain, However, it is clear that more than about half of the 600 connecting moieties may well react with a labelling species, and the number of labelling species may thereofre be higher than about 400.

The number of labelling species and targeting species in a single polymeric carrier molecule according to the invention influences the minimum amount of biological cells including virus particles that can be detected according to the invention. In one embodiment the minimum amount of cells detectable is less than 2000 cells per microlitre sample specimen, such as less than 1900 cells per microlitre, for example less than 1800 cells per microlitre, such as less than 1700 cells per microlitre, for example less than 1600 cells per microlitre, such as less than 1500 cells per microlitre, for example less than 1400 cells per microlitre, such as less than 1300 cells per microlitre, for example less than 1200 cells per microlitre, such as less than 1100 cells per microlitre, for example less than 1000 cells per microlitre, such as less than 900 cells per microlitre, for example less than 800 cells per microlitre, such as less than 700 cells per microlitre, for example less than 600 cells per microlitre, such as less than 500 cells per microlitre, for example less than 450 cells per microlitre, such as less than 400 cells per microlitre, for example less than 350 cells per microlitre, such as less than 300 cells per microlitre, for example less than 280 cells per microlitre, such as less than 260 cells per microlitre, for example less than 240 cells per microlitre, such as less than 220 cells per microlitre, for example less than 200 cells per microlitre, such as less than 180 cells per microlitre, for example less than 160 cells per microlitre, such as less than 140 cells per microlitre, for example less than 120 cells per microlitre, such as less than 100 cells per microlitre, for example less than 80 cells per microlitre, such as less than 60 cells per microlitre, for example less than 50 cells per microlitre, such as less than 45 cells per microlitre, for example less than 40 cells per microlitre, such as less than 35 cells per microlitre, for example less than 30 cells per microlitre, such as less than 25 cells per microlitre, for example less than 20 cells per microlitre, such as less than 15 cells per microlitre, for example less than 10 cells per microlitre, such as less than 5 cells per microlitre, for example less than 1 cell per microlitre sample specimen.

In another embodiment of the invention the mimimum amount of cells detectable is less than 1000 cells per millilitre, for example less than 900 cells per millilitre, such as less than 800 cells per millilitre, for example less than 700 cells per millilitre, such as less than 600 cells per millilitre, for example less than 500 cells per millilitre, such as less than 400 cells per millilitre, for example less than 300 cells per millilitre, such as less than 200 cells per millilitre, for example less than 150 cells per millilitre, such as less than 100 cells per millilitre, for example less than 90 cells per millilitre, such as less than 80 cells per millilitre, for example less than 70 cells per millilitre, such as less than 60 cells per millilitre, for example less than 50 cells per millilitre, such as less than 40 cells per millilitre, for example less than 30 cells per millilitre, such as less than 20 cells per millilitre, for example less than 10 cells per millilitre, such as less than 5 cells per millilitre, for example less than 3 cells per millilitre, such as less than 1 cell per millilitre sample specimen.

The biological cell including a virus particle present in a sample specimen such as e.g. a body fluid sample is preferably detected and recorded as a positive diagnostic result according to one method of the invention in less than about 20 minutes, such as less than 15 minutes, for example less than 10 minutes, such as less than 8 minutes, for example less than 7 minutes, such as less than 6 minutes, for example less than 5 minutes, such as less than 4 minutes, for example less than 3 minutes, such as less than 2 minutes, for example less than 1 minute, such as less than 45 seconds, for example less than 30 seconds, such as less than 15 seconds.

The preferred statistical quality parameters for the present invention when the test is capable of detecting an amount of cells as specified above within a predetermined time period may be summarised as follows:
Sensitivity: at least 80 %, such as at least 85 %, such as at least 90 %
Specificity: at least 80 %, such as at least 85 %, such as at least 90 %
Positive predictive value: at least 80 %, such as at least 85 %, such as at least 90 % Negative predictive value: at least 80 %, such as at least 85 %, such as at least 90 %, more preferably at least 99.5 %

The positive and the negative predictive value is closely related to the prevalence of the disease in the population, to be tested. In the present context it is preferred that the statistical calculations are based on a type of population that are realistic for using the test. Thus, the statistical calculations are not based only on a population known to have acquired the disease, but also on individuals that might turn out as negatives for the disease. Due to the validity and sensitivity of the kit according to the present invention the kit is particular suitable for testing of populations having a prevalence of the condition being tested for less than 100 %, such as less than 90 %, such as less than 80 %, more preferably less than 70 %, even more preferred less than 60 %, such as about 50 %.

The present invention provides in another embodiment a method for detection of biological cells including viruses in a specimen, wherein said specimen, optionally treated to remove undesired components, is contacted with an extended solid phase having conjugated thereon a targeting species, preferably an antibody, directed against the biological cell including a virus. The contacting results in the case of an antibody being used in the formation of immuno-complexes with antigens characteristic of the biological cells and viruses to be targeted.

The extended solid phase is separated from the specimen; said separated extended solid phase is contacted with a mobile solid phase comprising a polymeric carrier molecule according to the invention having conjugated thereto a predetermined tergeting species such as an antibody. The antibody results in the binding of said polymeric carrier molecules according to the invention to said immuno-complexes; the extended solid phase is subsequently separated from said mobile solid phase; and the presence of polymeric carrier molecules according to the invention bound to said extended solid phase is detected, whereby the presence of biological cells including viruses in said specimen is detected or determined.

Also, the invention provides a biological cell detection kit and a virus detection kit which comprises as individual components: (a) an extended solid phase having conjugated thereon a targeting species, preferably an antibody capable of forming immuno-complexes with antigens characteristic of the biological cells and viruses to be detected; and (b) a mobile solid phase consisting of dispersed polymeric carrier molecules according to the invention having conjugated thereto said targeting species, or a different target species, preferably an antibody, characteristic of the biological cells and viruses to be detected.

A specimen which may comprise a biological cell including a virus of types to be detected is in one embodiment exposed to an extended solid phase component which is coated at least in one location with a targeting species which will form complexes with the antigens of the biological cells and vira to be detected.

The extended solid phase is in one embodiment separated from the specimen, such as by washing the specimen off the extended solid phase, and the separated extended solid phase is then contacted with a mobile solid phase of dispersed polymeric carrier molecules according to the invention comprising the same or different targeting species, preferably an antibody. If immuno-complexes of antigens of biological cells including vira to be detected ("target" biological cells and "target" vira, respectively) have formed on the extended solid phase, the polymeric carrier molecules according to the invention will be bound to such complexes.

The unbound polymeric carrier molecules according to the invention of the mobile solid phase then are removed, such as by washing, and the extended solid phase is examined to determine the presence of polymeric carrier molecules according to the invention bound to the extended solid phase. These may be visually detected in some cases, for example when the polymeric carrier molecules according to the invention have been initially stained or dyed. Microscopic examination may be employed. The use of tracers or labels for the polymeric carrier molecules according to the invention enables the use of other detection methods as described herein below in more detail.

By this means, the presence or absence of bound polymeric carrier molecules according to the invention enables detection of the presence or absence of the target biological cells including vira, and an evaluation of the quantity of bound polymeric carrier molecules according to the invention enables determination of the quantity of biological cells including vira in the specimen, for example by comparison with standard results for the assay of known samples.

The extended solid phase used in the present invention may be employed in a variety of forms or structures. The solid phase has a location where a targeting species, preferably an antibody, can bind or associate, and the formation of such a solid phase with said targeting species, preferably an antibody, enables contacting a specimen and other materials used in the method of the invention. Preferred specimens are body fluid samples as described in more detail herein below.

The extended solid phase is best formed in a way which enables simple manipulation for easy contact with the specimen and other reagents. For this purpose, the extended solid phase may form at least part of a dipstick, syringe, tube or container.

The specimen and other reagents can be drawn in and ejected from a syringe, caused to flow through a tube, or deposited in a container such as a test tube shaped container. In such devices, the extended solid phase can form the whole of the device, or part of it, where, in the case of a syringe, tube or container, the part formed of the extended solid phase will at least be exposed at the inside of the device to permit contact with specimen and reagents. Targeting species, preferably an antibody, is preferably concentrated at one location of the extended phase, to be exposed to the specimen.

One more preferred form of the extended solid phase is a dipstick. In such a dipstick, it is further preferred that the extended solid phase should be included at at least one end, and that the targeting species, preferably an antibody, conjugated on the extended solid phase should be concentrated at the end of the dipstick. The extended solid phase can however comprise the entire dipstick, with the targeting species, preferably an antibody, concentrated at one end, or in more than one location.

The dipstick may be entirely formed from the extended solid phase, at one end of which has been conjugated a coating of targeting species, preferably an antibody. In another embodiment the dipstick has an extended solid phase one end of which is adhered to a body portion. A coating of targeting species, preferably an antibody, is conjugated to the extended solid phase. In yet another embodiment the extended solid phase entirely forms a tubular container into which a specimen can be placed. Coatings of targeting species, preferably an antibody, are located near the bottom of the container and are concentrated in one or more locations.

The extended solid phase is composed of any material onto which the desired targeting species, preferably an antibody, can be effectively bound. For covalent binding with antibody protein, the solid phase material can be chosen to contain a functional carboxyl surface, with use of a water-soluble carbodiimide as a conjugation reagent. A preferred material is acrylic resin, which has a carboxylated surface that enables binding the desired targeting species, preferably an antibody, by conjugation. For materials with amino surface groups, reactive carboxyl intermediates can be prepared by reacting with succinic anhydride. A variety of inorganic supports, typically glass, can also be prepared for covalent coupling with targeting species, preferably an antibody. Reference is made, for example, to "Enzymology, A Series of Textbooks and Monographs," Vol, 1, Chapter 1, 1975.

Extended solid phase materials capable of binding targeting species, preferably an antibody, are selected from materials which do not cause serious interference with the assay steps.

The presence of non-specific agglutinators in a tissue specimen, particularly those coupled to immunoglobulins, can result in error by causing the binding of mobile polymeric carrier molecules according to the invention to the extended solid phase even in the absence of specific Ag. Repeated washes during the assay would reduce the non-specific binding, but removal of the non-specific agglutinators is necessary in order to avoid such undesired binding. A simple polystyrene latex surface, for example, can passively delete some of the agglutinators, whereas an Ig G-coated surface provides a better affinity.

For convenience in the following description, the extended solid phase will be referred to as the preferred dipstick, although other forms may be used as explained herein above.

In one embodiment the biological cell essentially consists of or comprises a viral particle. A typical viral particle has an envelope of many, usually over one hundred, identical antigenic epitopes or protein sets. The proteins provide very strong binding with specific antibodies and form multiple conjugates or immune complexes. Highly specific antibodies in monoclonal form are also available, either produced by hybridoma for the selected monoclonal mouse antibody, or by the human B-lymphocytes transformed by the Epstein-Barr virus for the human IgM.

When properly chosen, these monoclonal antibodies can provide consistant and reproducible binding with virions. With a proper supply of specific antibody, the present direct binding immunoassay, in contradistinction with competitive binding immunoassay practiced in radioimmunoassay, can be a reliable and very rapid procedure since the incubation time for a kinematic equilibrium needed in competitive binding assays is not presently required.

In accordance with the method of the present invention, antiviral antibody targeting species, either from the usual Ig fraction of the antisera or from monoclonal antibodies, is conjugated respectively with a solid phase dip stick as well as with a mobile solid phase, or the so called "monodispersed" polymeric carrier molecules according to the invention.

The functions of the dip stick are for the handling and the separation of bound from free antigens, whereas that of the mobile polymeric carrier molecules according to the invention are for the detection of the formed immuno-complexes. Coupling techniques between the antibody protein and various solid phase materials are well developed (see, for example, the above-mentioned W. J. Dreyer, U.S. Pat. No. 3,853,987).

In one embodiment of the method of the present invention described above, the resulting immunocomplex is a multilayered "sandwich" comprising:

Dip stick + targeting species, preferably an antibody + viral antigen + targeting species, preferably an antibody + polymeric carrier molecule comprising a labelling species.

The amount of antibody required for covalent binding, however, can be less than a thousand times that of passive adsorption to a plastic such as polyvinyl chloride and the economics of using such an amount of highly specific targeting species, preferably an antibody, can be prohibitive.

An alternative way of binding that retains some strength of the covalent binding as well as the specificity of targeting species, preferably an antibody, is to bridge the targeting species and the solid phase with a first antibody, an antispecies antibody targeted against the Fc portion of the targeting antibody. Such an Fc portion is illustrated e.g. in "Immunology" (1981), The Upjohn Company, Kalamazoo, Mich.

That is, an inexpensive first antibody may initially be covalently bound to the solid phase, and the bound first antibody attracts the species-specific Fc portion of a targeting antibody, leaving the functional epitope of the targeting antibody unaltered with regard to an antigen of a biological cell or a viral particle. Bridged with such a first antispecies antibody, the immunoassay of the present invention brings about the following coupling "sandwich" in the case of detection of a viral species:

Dip Stick + antispecies antibody + targeting antibody + viral antigen + targeting antibody + antispecies antibody + polymeric carrier molecule comprising a labelling species.

In the direct binding assay of the present invention, the couplings between the dipstick and targeting species, preferably an antibody, as well as the couplings between the polymeric carrier molecules according to the invention and targeting species, preferably an antibody, are prepared in advance, and elements of non-specific agglutination in the fluid specimen are removed or deactivated for pretreatment prior to the direct binding assaying as mentioned above.

The assaying procedure according to one embodiment of the invention is therefore simplified to the following steps:
(1) Insert the dipstick into an optionally pretreated specimen in the form of a body fluid sample.
(2) Incubate dipstick and sample.
(2) Wash the dipstick.
(3) Insert the dipstick into a dispersion of polymeric carrier molecules comprising at least one targeting species, preferably an antibody, and at least one labelling species, unless the polymeric carrier molecules have previously been added to the specimen.
(4) Optionally wash the preparation obtained under (3).
(5) Detect the polymeric carrier molecules according to the invention on the dipstick by detecting either the targeting species or the labelling species.

In order to use a minimal amount of wet chemistry, the present detection of attached polymeric carrier molecules according to the invention on a dip stick is made independent of the immune chemistry. By concentration of the targeting species, preferably an antibody, at one end of the dipstick, the bound polymeric carrier molecules according to the invention are concentrated at one location, which simplifies detection.

The polymeric carrier molecules according to the invention can include any dye or fluorescent compound for direct visual observation, or have metal elements or iron oxide doped or entrapped within in order to provide X-ray fluorescent or electromagnetic signals. Enzymatic amplification can also be designed into the polymeric carrier molecules according to the invention.

In one embodiment, the present method employs a direct binding assay instead of a competitive binding assay where a dynamic equilibrium necessitates lengthy incubation. The disclosed method can, of course, be employed in a competitive protein binding assay as well. The roles of the immune analytes antibody and antigen can also be interchanged, still making use of the immobilized solid phase for the signal amplification. Binding of antibody or various antigen molecules to the solid phase matter is well known, in passive adsorption as well as in covalent coupling.

In the immunoassay of the present invention, the antigen characteristic for a biological cell or a virus, which optionally appears in high multiplicity, is used as a bridge to connect the mobile and the immobilized solid phases. This connection can obviously also be served by various other antigens with multiple antibody binding sites. In cases of certain antigen without repetitive binding sites which cannot specifically connect more than one monoclonal antibody, polyvalent antibodies may also be used instead.

The method of the invention can also be designed to assay several analytes in a single procedure where each analyte is represented by a particular pair of corresponding binding partners including antibodies, antigens, and the same or different polymeric carrier molecules comprising one or more targeting species.

Detection of different types of biological cells including vira can be done in accordance with the invention by conjugating a plurality of different targeting species, preferably an antibody, proteins capable of forming complexes with corresponding antigens of different biological cells including vira, respectively to the extended solid phase and to the mobile solid phase. The visual observation or other detection of any bound microspheres following the assay indicates that one or more of the different biological cells including vira is present in the specimen, and this assay, if positive, can be followed by assays for individual biological cells including vira of the different ones which were tested for simultaneously.

In another embodiment, the different biological cells including vira can be both simultaneously and individually detected. For such a test, the different targeting species, preferably an antibody, corresponding to the antigens of a plurality of different types of biological cells including vira are conjugated to microspheres which are correspondingly labelled with different metal elements.

When more than one type of the differently labelled microspheres are bound to the extended solid phase in the assay of the invention, they may be separately and simultaneously detected. In this way, the presence of corresponding individual types of biological cells including vira in the specimen are simultaneously and separately detected. This is particularly relevant when determining subtypes of vira including subtypes of a respiratory syncytial virus.

The extended solid phase and the dispersed microspheres which are conjugated with targeting species, preferably an antibody, prepared as described above as individual components useful for the assay method of the invention, can be provided in the form of a virus detection kit comprising such components. Different kits may be provided, which differ as to the targeting species, preferably an antibody, coatings, and thus as to the vira to be detected.

Such a kit may further include as an individual component, a latex solid phase for removing non-specific agglutinators from a specimen prior to the assay. The preferred latex for this purpose is polystyrene coated with gamma immunoglobulin.

The extended solid phase and mobile solid phase components of the kit of the invention may be provided with targeting species, preferably an antibody, bound to an antispecies antibody as disclosed above. Also as disclosed above, the microsphere component may be labelled, and the extended solid phase can take the form of part or all of a dipstick, syringe, tube or container, coated with targeting species, preferably an antibody, in at least one location, as disclosed.

Furthermore, the extended solid phase component may be provided with a plurality of different targeting species, preferably an antibody, capable of forming complexes with corresponding antigens of different types of biological cells including vira. When it is so provided, the individual mobile solid phase component can be provided either to have the same plurality of targeting species, preferably an antibody, conjugated to each of the polymeric carrier molecules thereof, or a mixture of different types polymeric carrier molecules can be provided, each type having conjugated thereto a different targeting species, preferably an antibody, of said plurality; or in a further variation, the mobile solid phase component can be provided in the form of separate batches of polymeric carrier molecules, each batch having conjugated thereto a different targeting species, preferably an antibody, of said plurality.

Although it is preferred to use a polymeric carrier molecule comprising a targeting species and a labelling species as described herein above, the invention can also be exercised by using spherical particles including microspheres comprising a targeting species and optionally also a labelling species. Such microspheres may in particular be used in connection with a micro system comprising the kit according to the invention.

The kit according to the invention may also be applied in a micro system, such as a micro flow system described in WO 98/10267, one such system being marketed by Torsana Biosensor A/S, Denmark.

The principle behind the technology of a micro flow system is that by controlling the flow rate of at least two guiding streams, a sample stream can be accurately positioned on a target surface.

By controlling the flow ratios between the guiding streams and the sample stream, the sample stream can be focused to a width of a few mm. The sample stream carries the molecules to interact with the surface.

Immobilized lanes of the system are interacted with liquid streams containing unknown samples in the y-dimension.

Thousands of unique intersection points are created where reaction can occur.

The fact that no turbulence occurs in very narrow fluid streams results in diffusion being the only phenomena perturbing the focus of the sample stream. In effect, the technology permits the precise positioning of a liquid stream on a planar surface. In this way it is possible to position material with a precision of a few mm.

The microfluidic system allows for control of very narrow streams of liquid carrying the material (DNA, proteins, cells) to be interacted with the surface of the chip.

The micro flow system enables immobilization of reactant streams, in the present context streams of conjugate comprising the polymeric carrier and subsequent testing with one or several samples creating a weave with thousands of intersection points where chemical reactions occur and are detected. The entire procedure is performed in a closed fluidic system providing the flexibility in terms of sample - and reactant application, choice of immobilization - and detection chemistries, and array layout.

In particular when using the kit for testing for a plurality of biological cells and/or inflammatory indicators, such as providing profiles of inflammatory indicators, the invention suitably includes the use of the kit in a microsystem.

In addition to micro systems, the kit according to the invention may also form part of a conventional macro system such as e.g. a lateral flow device. Examples of such devices are listed herein below.

United States Patent 5,610,077 (Unilever) discloses a process for performing a specific binding assay. Accordingly, there is provided a method according to the invention for carrying out a specific binding assay comprising the steps of reacting (a) a sample under assay comprising a biological cell with (b) a specific binding partner for the biological cell being tested for, including a polymeric carrier molecule according to the invention, immobilised on a solid support, and (c) a specific binding partner for the biological cell being tested for, including a polymeric carrier molecule according to the invention, which is conjugated to a detectable marker, thereby to form a sandwich complex by reaction between whatever quantities are present of the biological cell being tested for with reagents (b) and (c) and immobilising the marker to the support via the biological cell being tested for, the marker being detected or assayed as an index of the quantity of the biological cell being tested for present in the sample (a), the improvement which comprises using reagents (b) and (c) together for reaction with sample (a) and avoiding competitive interference between the binding reactions of the biological cell being tested for and reagents (b) and (c) by using as reagents (b) and (c), monoclonal antibodies each of narrow and different, non-interfering specificity, the binding reagent (b) being immobilised on the surface of a displacer body which occupies a majority of the volume of a well or cup containing aqueous liquid in which the specific binding reaction takes place.

The narrow specificity required of the antibody is a capacity to bind specifically with the biological cell under test but without preventing the binding reaction between the biological cell under test and its other specific binding partner. The conjugate between antibody and the enzyme or other marker, and/or the antibody (if any) which is coupled to the solid surface, may comprise a monoclonal antibody or other antibody of sufficiently narrow specificity to ensure that the desired assay reaction or reactions are not impeded by competition between the conjugate and the immunosorbent in their reactions with whatever quantities are present of the biological cell being tested for in the sample under assay. An antibody of sufficiently narrow specificity may also be obtained in the (polyclonal) immunoglobulins of antisera raised against discrete chemical or physical molecular fragments of the material under test, for example, antibody against Fc fragments (or against smaller peptide fragments) of immunoglobulins to be tested for, or against sub-units or peptides of protein antigens to be tested for. The object in each case is to ensure substantial freedom from interference which can arise particularly, for example, in carrying out immunoassays of the "sandwich" or "antiglobulin" test configurations.

In a "sandwich" test configuration, antigen under test can be specifically adsorbed to a first antibody bound to a solid surface, and a second antibody carrying an enzymic or other (e.g. fluorescent or radioactive) marker is specifically bound to the adsorbed antigen under test. Marker specifically so bound is used for measurement and determination of the antigen under test, e.g. by direct measurement, such as radiometry or fluorimetry, or exposure of enzyme marker to substrate followed by product measurement. Thus, in preferred sandwich tests, the two antibodies used can have different, non-interfering specificity with respect to the same antigen under test.

In an "antiglobulin" test configuration, sometimes also referred to as a "sandwich" test configuration, the position is analogous: the material under test is itself an immunoglobulin; the material bound to a solid surface is its corresponding antigen or hapten; and the material carrying the marker is an antiglobulin corresponding to the species and immunoglobulin type of the antibody under test. In preferred antiglobulin tests, the antiglobulin can have sufficiently narrow specificity as not to interfere with the subsequent adsorption of its corresponding globulin to the insolubilised antigen.

If antibodies from ordinary antisera raised against unmodified antigen (polyclonal antibodies) are used in sandwich or antiglobulin tests, there is a very likely risk that if all ingredients are mixed in a single step there will be interference between the two specific adsorption reactions. When such tests are carried out according to the present invention, using apparatus as described herein, such interference can be avoided either by using antibodies of narrow specificity as described, or else by ensuring that the binding of test material to the solid surface takes place before exposure of test material to the other (marker-conjugated) binding agent if there is a risk that binding by that other agent would prevent subsequent adsorption to the solid surface. Such a sequence can be ensured by arranging for slow release of the other (marker-conjugated) binding agent.

Particular instances of suitable assay specificities, antibody specificities, and slow-release forms of conjugated reagent (c) are described for example below.

It has also been found that in carrying out such specific binding assays, a worthwhile improvement in reaction kinetics can be obtained if the reaction liquid containing ingredients (a), (b) and (c) is contained in a well or cup of which the majority of the volume is occupied by a displacer body. (The use of inserts of various rod or ball shaped forms is known in connection with other kinds of immunoassay, as described in G. B. Specification Nos. 1,414,479 and 1,485,729.)

The displacer body can, for example, be of a shape substantially complementary to and slightly smaller than that of the cup or well, so that the liquid phase containing one of the specific binding reagents is approximately in the form of a shell occupying the space between the displacer and the cup or well. The displacer can be loose-fitting and not fixedly mounted, i.e. movable relatively to the cup or well, so that by relative motion between displacer and well the liquid between them can be given a stirring or agitation motion.

For example, a round well can have a round displacer therein with an external diameter slightly smaller than the diameter of the well. The presence of the displacer can reduce the space available for liquid in the well by a factor of for example 2-10, e.g. 3-8, comparing volumes based on similar liquid levels in the well, e.g. when filled to its normal operating level, or its maximum capacity. For example, a microtitre well designed to have 300 microlitre of liquid filled into it during a normal assay, can be used with a displacer leaving 30-150 microlitre liquid space, e.g. 50-100 microlitre.

The use of wells or cups together with displacers as described herein can improve the efficiency of the assay reaction steps because, in the first place, it allows more concentrated reagents to be used with no increase in the weight of reagent or decrease in the size of the microtitre wells, compared with the normal conditions encountered in microtitre wells of given size; and in the second place, it increases the sensitised surface area available to react with a given liquid reagent volume, so that comparatively faster adsorption kinetics can be achieved without having to increase specific reagent density on the sensitised surface or encountering problems of crowding.

A set of displacer bodies can be preferably present in certain embodiments of the invention, e.g. as an integral part of a lid which can be fitted onto a microtitre plate, e.g. a standard plate of 8 times 12 wells. The set can be large enough to fit all wells of the plate or a sub-set thereof, e.g. a row. The dimensions of the displacers and the volume of liquid to be dispensed into the well can be chosen relative to the well in the manner described above, and preferably so that the liquid to be tested is in contact with substantially the major part and preferably the whole inner surface of the well.

The immobilised specific binding partner (reagent (b) for the biological cell to be assayed can be immobilised on the wall of the well or cup in which the assay reaction takes place. Alternatively, according to a feature of the invention independently capable of providing advantage and convenience in use, a liquid displacer, for example in the form of a stick, peg or stud, for dipping into a liquid assay reagent, can have an immunosorbent surface. This allows the portion of the assay materials needing to be carried over from one reagent to the next, and the associated manipulations, to be handled more easily than when the sensitised surface is part of a hollow well. An alternative form for such a liquid displacer body is a tuft of bristles or leaves of suitable material, or equivalent body with large surface area. A further alternative form is a stud or peg with relatively hollowed-out and projecting portions of its surface, e.g. with grooves and associated ribs, e.g. annular grooves. Such an arrangement can give robustness, increased sensitised surface area, and better reactivity.

Test apparatus according to related embodiments of the invention can thus comprise a set of sensitised liquid-displacer bodies of one or more of such forms, joined to a common handling-bar, link or lid, and for use in combination with a complementary set or sets of wells containing any of the remaining materials used in the assay. The several displacer bodies of the set can have the same or different sensitisation so that one or a plurality of different assay types can be carried through simultaneously. If desired, the displacer bodies can be removably and exchangeably mounted on the handling bar, link, or lid, so that sets of desired specificity can be built up at will from a common stock for carrying out large numbers of tests according to a desired pattern.

One advantage of such arrangements is that a number of displacer bodies can be sensitised in the same body of liquid ragent, avoiding fluctuating conditions of concentration, etc., resulting from dosing aliquots into wells.

The displacer bodies can, in this embodiment, be of any material suitable for the preparation of an immunosorbent by covalent bonding or adsorption: e.g. polystyrene, nylon, or cellulose acetate. (The nature of the displacer surface does not matter provided it is inert, when the sensitisation is to be on the well surface rather than the displacer surface.) Linkage of antibodies, antigens, etc., to the displacer bodies can be carried out by linking methods known in themselves, e.g. partial acid hydrolysis of nylon surface, substitution of exposed amine surface with glutaraldehyde, and coupling of material to be bound, e.g. antibody or antigen to immobilised aldehyde groups. Suitable methods among a wide variety are given for example by Inman & Hornby (1972) Biochem. J. 129, 255; Campbell, Hornby & Morris (1975) Biochim. Biophys. Acta 384, 307; Mattiasson & Nilsson (1977) F.E.B.S. Letters 78, 251; and G.B. patent specifications Nos. 1,470,955 and 1,485,122.

It can be seen that the invention also provides a kit of test materials for carrying out a specific protein-binding assay, comprising (i) an immobilised specific binding partner for a biological cell to be tested for, including a polymeric carrier molecule according to the invention, carried on a solid support, and (ii) a marker-conjugated specific binding partner for the biological cell to be tested for, including a polymeric carrier molecule according to the invention which can be added to a reaction liquid contacting immobilised reagent (i) either as a slow-release form, or in any form provided that the specific binding partners in reagents (i) and (ii) include an antibody of narrow specificity so that reagents (i) and (ii) do not interfere with each other's binding reactions with the biological cell to be tested. Optionally the kit can also comprise materials for later estimation of the amount of marker immobilised during the assay.

Reagent (i) can be immobilised on either a displacer body for a reaction well, or on a reaction well wall, as described above. A slow-release form of reagent (ii) can be for example a sucrose or equivalent glaze on a complementary surface of either the displacer or the well wall, also as described above. The narrow-specificity antibody can be selected for example from monoclonal antibodies in the manner already described.

United States Patent 5,501,949 (Murex) pertains to a method for detection or quantitation of an analyte in a solution. Accordingly, the present invention in one embodiment pertains to a method comprising the steps of:
(a) contacting the solution with insoluble particles or a polymeric carrier molecule according to the invention having attached thereto a binding component specific for the analyte, so as to form a suspension comprising a first complex wherein the first complex comprises the analyte, the binding component and the insoluble particle;
(b) applying the suspension to at least a portion of a semi-permeable membrane having interstices of dimensions relative to the insoluble particles or the polymeric carrier molecules according to the invention and having a thickness such that the insoluble particles or the polymeric carrier molecules according to the invention are retained throughout the thickness of the semi-permeable membrane, the portion of the semi-permeable membrane retaining the particles or polymeric carrier molecules defining an assay zone;
(c) contacting the semi-permeable membrane containing the assay zone with a labeling component, preferably comprised in a particle or a polymeric carrier molecule according to the invention and forming part thereof, said particle or polymeric carrier molecule being capable of specifically binding to the first complex so as to form a second complex wherein the second complex comprises the first complex including the labeling component, wherein the semi-permeable membrane permits the labeling component which is not bound to the first complex to pass out of the assay zone; and
(d) measuring the signal produced by the labeling component of the second complex as an indicator of the presence or amount of the analyte present in the solution.

United States Patent 5,155,021 (EASTMAN KODAK) discloses a diagnostic kit useful for the determination of a herpes simplex virus. Accordingly, the present invention in one embodiment relates to a diagnostic kit comprising:
i) polymeric particles which are substantially free of chemical or biological materials, having an average diameter of from about 0.01 to about 10 micrometers, and which have a surface area of from about 0.1 to about 600 m²/g of particles, which particles are capable of having herpes simplex viral antigen directly bound thereto,
ii) a disposable test device comprising a microporous membrane which has an average pore size of from about 0.1 to about 20 µm, and
iii) antibodies which bind to a biological cell according to the invention.

The antibodies may be labeled with enzyme or they may be unlabeled, in which case the kit further comprises labeled antibodies which bind to said biological cell. Test device comprises three test wells, each well having a microporous membrane prepared from a polyamide mounted therein. Polymeric particles are supplied on the microporous membrane of said test device - particles are supplied in an aqueous suspension. The microporous membrane may be any suitable membrane, for example selected among the following commercially available pads
Whatman GF/D
Whatman F147-11
Whatman GF/AVA
Whatman 147-02
Whatman GF/DFA
Whatman F147-09
Whatman F075-17 *
Millipore Rapid Q24 *
Millipore Rapid Q27
Ahlstrom A142

Furthermore, an absorbant pad may be provided to absorb the liquid from the aqueous suspension. Absorbant pads are for example
Whatman D28
Whatman 1.5WF *
Whatman 3MM CHR

Further examples of assay devices and diagnostic methods pertaining to the invention includes, but are not limited to:

An assay device preferably comprising:
i) a zone for applying a body fluid sample comprising an indicator of infection and/or inflammation, said zone comprising at least one movable reporter species capable of binding said indicator, said application zone being in liquid contact with
ii) a zone for detecting the presence, amount or concentration of said at least one reporter species bound to said indicator, said zone further comprising a binding species for immobilizing onto said detection zone at least a substantial amount of said indicator comprised in said body fluid sample, and optionally
iii) a positive control zone generating a positive control confirming the transfer of at least part of said body fluid sample from said application zone to said detection zone.

The at least one reporter species comprised in the sample application area preferably comprises an antibody comprising at least one tag, linker or marker that makes it possible at least to detect the presence of said marker, and preferably also makes it possible to quantifiably detect said antibody and/or said reporter species bound to said indicator.

The binding species of the detection zone is preferably also an antibody, but this antibody may not comprise any tag, label or marker. It is thus possible to immobilise onto the detection zone an amount of a quantifiably detectable reporter species that accurately reflects the amount of marker present in the body fluid sample. The at least one tag, label or marker used preferably allows both visual detection, by means of the generation of e.g. electromagnetic radiation or a visible colour, and quantification of e.g. the emitted electromagnetic radiation.

Movable reporter species shall be understood to comprise a reporter species capable of moving on e.g. a solid or semi-solid surface, e.g. when being applied to a lateral flow device.

In one embodiment of this aspect of the invention there is provided an assay device for detecting an indicator of infection and/or inflammation present in a body fluid sample, said device comprising:
i) a hollow casing having a body fluid sample application aperture and a test result observation aperture,
ii) a bibulous body fluid sample receiving member within said hollow casing to receive said body fluid sample applied to said sample application aperture,
iii) a test strip comprising a dry porous carrier such as nitrocellulose within said casing and extending from said bibulous body fluid sample receiving member to and beyond said test result observation aperture, said dry porous carrier having a test result zone observable through said observation aperture,
iv) at least one of said bibulous body fluid sample receiving member and said test strip containing upstream from said test result zone a detectable reporter species capable of specifically binding said indicator to form a first complex,
v) said reporter species comprising at least one particulate label, such as a dye sol, a metallic sol or a coloured latex particle, and optionally also at least one fluorescently detectable label, said label being released into a mobile form by said body fluid sample,
   wherein mobility of said label within said test strip is facilitated by either coating at least a portion of said test strip upstream from said test result zone with a material comprising a polysaccharide, or drying said label onto a portion of said test strip upstream from said test zone in the presence of a material comprising a polysaccharide, in an amount effective to reduce interaction between said test strip and said label, and
   wherein said dry porous carrier contains in said test result zone a means for binding said first complex, said means for binding comprising specific binding means immobilized in said test result zone, and
   wherein migration of said body fluid sample from said bibulous sample receiving member into and through said dry porous carrier conveying by capillarity said first complex to said test result zone of said dry porous carrier whereat said binding means binds said first complex thereby to form a second complex, and
vi) determining the presence, amount or concentration of said second complex being observable through said test result observation aperture.

In another embodiment there is provided an assay device for detecting an indicator of infection and/or inflammation in a body fluid sample, said device comprising a solid support including at least one detectable reporter species on a test area of the solid support, said at least one detectable reporter species being capable of binding said marker, said reporter species further comprising a liposome or a microcapsule comprising a visible particulate dye compound and optionally also a fluorescently detectable marker.

In yet another embodiment there is provided an assay device comprising
i) a sample application area comprising a predetermined amount of a reporter species comprising an antibody capable of binding said indicator deposited thereon, said area being in fluid communication with
ii) a reaction zone comprising a mobilizable reporter species comprising an antibody capable of binding said indicator, said reporter species further comprising at least one visually detectable particle and/or at least one fluorescently detectable particle, and
iii) a detection zone comprising a reporter species comprising an antibody capable of binding said indicator,
wherein, when said body fluid sample comprising said indicator is applied to said sample application area, a threshold amount of the indicator is bound to said antibody and thereby prevented from binding to the antibody being present in the reaction zone, and
wherein the indicator remaining unbound in said body fluid sample passes from the sample application area through said reaction zone, where it is bound to said mobilizable reporter species comprising i) an antibody capable of binding said indicator, and ii) at least one visually detectable particle and/or at least one fluorescently detectable particle, and
wherein the indicator bound to the mobilizable reporter species is brought into contact with the detection zone, where the indicator is bound to said reporter species comprising said antibody capable of binding said indicator, and
wherein said binding of said indicator results in immobilization of said mobilizable reporter species further comprising i) an antibody capable of binding said indicator, and ii) at least one visually detectable particle and/or at least one fluorescently detectable particle,

The present invention pertains to detection of any biological cell including any viral particle. Vira to be detected manually or by use of an automated system including a microsystem includes, but are not limited to, Herpes Simplex vira in various organs, particularly from cervical PAP smears or cerebrospinal fluid; Cytomegalovira (CMV) in urine, kidney, lung and brain; Varicella-Zoster vira in the brain; Cox-Sackie B group vira in the heart; Measles vira in lymph node and lung; Respiratory syncytial virus in nasal secretions and in the lung; Hepatitis B vira in serum; Hepatitis A vira in stool, and the like.

As the clinical goal of a viral assay according to the invention is often the absence of the analyte of interest, and not its amount, several viral analytes can therefore be combined into a single test. For example, from a lung tissue, the antibody conjugated polymeric carrier molecules can include the tests for Herpes vira, Cytomegalovira, and Respiratory syncytial vira. In the presence of a positive result, the test may proceed further for a specific and quantitative identification.

In one embodiment of the present invention the biological cell to be detected manually or by use of an automated system including a microsystem is a picorna virus particle. Picornaviruses are a family of very small non-enveloped viruses having a core of capsid-enclosed positive-stranded RNA. The picornaviruses infect a broad range of animals, including humans, cattle and swine. Examples of picornaviruses include rhinoviruses, enteroviruses for example poliovirus, coxsackievirus and echovirus, cardioviruses, for example encephalomyocarditis virus and meningovirus, aphthoviruses for example foot-and-mouth disease virus, as well as hepatistis A virus.

In one preferred embodiment the biological cell is an aphtovirus, more preferably a foot-and-mouth diesease virus. Preferably such a foot-and-mouth disease virus is selected from the group consisting of foot-and-mouth disease viruses with the serotype: A, O, C, SAT1, SAT2, SAT3, Asia1.

Such aphtoviruses could be detected in variety of host animals, preferably domestic animals which could for example be selected from the group consisting of: cattle, domestic buffaloes, yaks, sheep, goats and swine. Samples could be derived from various organs, such as lymph nodes, bone marrow or mucsle or from body fluid samples such as saliva, faeces, urine, milk, semen or blood samples.

In one particularly interesting embodiment the present invention is concerned with a method for detecting Respiratory syncytial virus (RS virus), or subtypes thereof, and a kit for use in such methods. The RS virus may be detected in any body fluid, including nasal secretions, and in the lung.

Two different subtypes of RSV (A and B) have been demonstrated for the first time in 1985 based on analyses of serologic antigenic variation using antibodies respectively directed against the large glycoprotein (G), fusion protein (F), matrix protein (M), nucleoprotein (NP) and phosphoprotein (P) components of the long strain of RS virus.

Subtype A viruses reacted with all the antibodies, whereas subtype B viruses showed different epitope characteristics in four structural components. The number of altered epitopes were 5/6, 1/2, 2/6 and 1/6 in the G, F, M and NP components, respectively (Mufson MA et al., J. Gen Virol 1985 Oct; 66 (pt10):2111-24).

Peptides deduced from the central conserved region (residues 158 to 189) of the G protein of human respiratory syncytial virus (HRSV) subtypes A and B have been used as antigens in subtype-specific enzyme-linked immunosorbent assays (G-peptide ELISAs) (Langedijk, JP et al., J Clin Microbiol 1997 Jul, p1656-1660.

All the above-mentioned antigenic determinants are suitable for detecting RS virus, or subtypes thereof, in accordance with the present invention.

A colorimetric microtiter plate (MTP) PCR system for specific detection of the respiratory syncytial virus (RS virus) nucleocapsid gene and differentiation of viral subtypes A and B has been developed for clinical laboratory diagnosis and simultaneous subgroup classification of RSV infection (Tang, YW et al., Diagn Microbiol Infect Dis 1999 Aug; 34 (4) 333-7). Such a test may be used in combination with the present invention.

In one embodiment the present invention pertains to simultaneous and rapid detection of RSV and the prevailing subtype (A or B) by using a polymeric carrier molecule according to the present invention comprising at least one targeting species characteristic for RS virus, including any subtype thereof, and at least one labelling species. Such a rapid detection of RS virus in nasal secretion is novel and does not require expensive laboratory equipment and time-consuming procedures.

When the present invention pertains to detection of RS virus, the polymeric carrier molecule may comprise any targeting species including antibodies capable of detecting RS virus including any subtype thereof (A or B, or otherwise). Preferred targeting species comprise antibodies such as e.g. the highly variable attachment protein G has limited homology between HRSV subtypes (53% amino acid homology). However, within the subtypes the amino acid homology is much larger: >80% within HRSV subtype A (HRSV-A) strains and >90% within HRSV-B strains.

Accordingly, protein G is a good candidate antigen for a discriminatory assay. The ectodomain of protein G contains a central, conserved, relatively hydrophobic region bounded by two hydrophilic, polymeric mucin-like regions. It is a major antigenic site, and peptides corresponding to this region can be used as antigens in immunoassays and probably as well in the invention in question.

Preferred antibodies for detecting RS vira according to the invention are selected from the below indicated list according to one embodiment of the invention:

Commercially available antibodies against human RSV (HRSV) virus (including subtypes A and B) suitable for use in the present application are for example as follows:

### Polyclonal antibodies:

| | |
|---|---|
| Biodesign International | Goat anti RSV (RSV-A and RSV-B) Specific for all viral antigens of RSV-A and RSV-B |
| Biogenesis | Recognises most RSV antigens |
| Fitzgerald Industries Int. | Fitzgerald Human RSV isolate, confirmed. |

### Monoclonal antibodies:

| | |
|---|---|
| Biodesign International | Specific for the fusion protein of RSV, types A & B. |
| Biodesign International | Specific for the fusion protein (F1 subunit). |
| Biodesign International | Immunogen: Purified RSV virus, strain Long |
| Biodesign international | Specific for the nucleoprotein of RSV including RSV-A and RSV-B |
| Biogenesis | RSV; Fusion protein |
| Biogenesis | This pool of antibodies reacts with RSV fusion and attachment glycoproteins, phosphoprotein and M2 proteins |
| Biogenesis | Recognises human isolate, all RSV antigens |
| Biogenesis | Recognises RSV glycoprotein molecular weight 89kD |
| Biogenesis | Recognises a 41/44 kD nucleoprotein |
| Chemicon Int. | Mab 92-11C specific for HRSV-A fusion protein 1b, and Mab 102-10B specific for HRSV-B fusion protein 1c |
| Fitzgerald Industries Int. | Specific for the fusion protein of RSV |
| Fitzgerald Industries Int. | Specific for the nuclear protein of RSV |

The present invention employs targeting species, labelling species, and more generally molecular species. The term "molecular species" in the context of the present invention is used to denote, for example: molecules or ionic species which serve as labels or markers (such as enzymes, or fluorescent or luminescent species); or molecules which serve as targetting species, i.e. molecules which are capable of binding selectively or specifically to one or more target molecules, moieties, receptors or epitopes (examples of such targetting species being haptens or hapten conjugates, antigens, antibodies, nucleotide sequences and hormones). The invention in one particular embodiment relates to simultaneously or sequentially using any one or both of a first targeting species and a second targeting species including polyclonal and monoclonal antibodies that may be, respectively, i) identical or non-identical, and ii) specific for the same or different epitopes of antigenic determinants characteristic for a biological cell according to the invention.

Molecular species according to the invention are to be found among numerous different types of substances, examples being: proteins, such as ferritin, phycoerythrins, phycocyanins or phycobilins; enzymes, such as horseradish peroxidase, alkaline phosphatase, glucose oxidases, galactosidases or ureases; toxins; drugs; dyes; fluorescent, luminescent, phosphorescent or other light-emitting substances; metal-chelating substances, such as iminodiacetic acid, ethylenediaminetetraacetic acid (EDTA), diethylenetriaminepentaacetic acid (DTPA) or desferrioxamine B; substances labelled with a radioactive isotope; or substances labelled with a heavy atom.

Many molecular species will be able to serve as labelling species in conjugates according to the invention. Additional examples of labelling species are listed herein immediately below.
i) Fluorescent substances selected from, e.g., fluorescein (suitably as fluorescein isothiocyanate, FITC), fluoresceinamine, 1-naphthol, 2-naphthol, eosin, erythrosin, morin, o-phenylenediamine, rhodamine and 8-anilino-1-naphthalenesulfonic acid.
ii) Radioactive isotopes of relevance may be selected, for example, among isotopes of hydrogen (i.e. tritium, ³H), carbon (such as ¹⁴C), phosphorus (such as ³²P), sulfur (such as ³⁵S), iodine (such as ¹³¹I), bismuth (such as ²¹²Bi), yttrium (such as ⁹⁰Y), technetium (such as ⁹⁹Tc), palladium (such as ¹⁰⁹Pd) and samarium (such as ¹⁵³Sm).
iii) Heavy atoms of relevance may be selected, for example, among Mn, Fe, Co, Ni, Cu, Zn, Ga, In, Ag, Au, Hg, I, Bi, Y, La, Ce, Eu and Gd. Gold (Au) may be used in combination with silver (Ag) as an enhancement reagent and Au is a particularly useful heavy atom in many cases.

Molecular species may also be in the form of targetting species capable of selectively binding to, or selectively reacting with, a complementary molecule or a complementary structural region of a material of biological origin. Examples of relevant targetting species are, for example: antigens; haptens; monoclonal or polyclonal antibodies; gene probes; natural or synthetic oligo- or polynucleotides; certain natural or synthetic mono-, oligo- or polysaccharides; lectins; avidin or streptavidin; biotin; growth factors; hormones; receptor molecules; or protein A or protein G. For examples of appropriate antibodies, reference is made to the working examples given herein. Examples of relevant hormones may be selected from steroid hormones (e.g. estrogen, progesterone or cortisone), amino acid hormones (e.g. thyroxine) and peptide and protein hormones (e.g. vasopressin, bombesin, gastrin or insulin).

The present invention may in one embodiment employ standard immunohistochemical or cytochemical detection procedures for the detection of the predetermined biological cell, or any suitable modifications of such procedures. Accordingly, the invention may employ any assay resulting in the recognition of an antigenic determinant mediated by an immunochemical reaction of the antigenic determinant with a specific so-called primary antibody capable of reacting exclusively with the target antigenic determinant in the form of a predetermined biological cell.

The primary antibody is preferably labelled with an appropriate label capable of generating - directly or indirectly - a detectable signal. The label is preferably an enzyme, an isotope, a fluorescent group or a heavy metal such as gold.

In another embodiment, the invention employ the detection of the primary antibody by immunochemical reaction with specific so-called secondary antibodies capable of reacting with the primary antibodies. In this case the secondary antibodies are preferably labelled with an appropriate label such as an enzyme, an isotope, a fluorescent group or a heavy metal such as gold.

In yet another embodiment, the present invention employs a so-called linker antibody as a means of detection of the predetermined biological cell. This embodiment exploits that the immunochemical reaction between the target antigenic determinant in the form of the predetermined biological cell and the primary antibody is mediated by another immunochemical reaction involving the specific linker antibody capable of reacting simultaneously with both the primary antibody as well as another antibody to which enzymes have been attached via an immunochemical reaction, or via covalent coupling and the like.

In yet another embodiment according to the present invention, the immunochemical reaction between a target antigenic determinant in the form of a predetermined biological cell and the primary antibody, or alternatively, between the primary antibody and the secondary antibody, is detected by means of a binding of pairs of complementary molecules other than antigens and antibodies. A complementary pair such as e.g. biotin and streptavidin is preferred. In this embodiment, one member of the complementary pair is attached to the primary or secondary antibody, and the other member of the complementory pair is contacted by any suitable label such as e.g. an enzymes, an isotope, a fluorescent group or a heavy metal such as gold.

A sample potentially containing a predetermined biological cell to be detected is preferably brought into contact with a polymeric carrier molecule comprising a labelled or non-labelled primary antibody capable of detecting said biological cell. The antibody becomes immunochemically bound to the predetermined biological cell comprised in the sample. The biological cell is then bound to a solid support containing the same or another labelled or non-labelled primary antibody capable of detecting said biological cell. When a lateral flow device is used, the labelled antibody bound to the predetermined biological cell is detected by reaction with appropriate reagents, depending on the choice of detection system.

The sample comprising the predetermined biological cell to be detected and optionally also quantified is in one embodiment of the invention subjected to at least one of the detection reactions described below. The choice of detection reaction is influenced by the targetting species in question as well as by the labeling species it is decided to use.

When an enzyme label is used as a labelling species, the biological cell bound to a solid support as described herein above is treated with a substrate, preferably a colour developing reagent. The enzyme reacts with the substrate, and this in turn leads to the formation of a coloured, insoluble deposit at and around the location of the enzyme. The formation of a colour reaction is a positive indication of the presence of the biological cell in the sample.

When a heavy metal label such as gold is used, the sample is preferably treated with a so-called enhancer in the form of a reagent containing e.g. silver or a similar contrasting indicator. Silver metal is preferably precipitated as a black deposit at and around the location of the gold. When a fluorescent label is used, a developing reagent is normally not needed.

It may be desirable to introduce at least one washing step after which some of the constituents of the sample are preferably coloured by reaction with a suitable dye resulting in a desirable contrast to the colour provided by the labeling species in question. After an optional final washing step, the specimen is preferably coated with a transparent reagent to ensure a permanent record for the examination.

Detection of the labeling species in question preferably indicates both the localization and the amount of the target antigenic determinant in the form of the predetermined biological cell. The detection may be performed by visual inspection, by light microscopic examination in the case of enzyme labels, by light or electron microscopic examination in the case of heavy metal labels, by fluorescence microscopic examination, using irradiated light of a suitable wavelength in the case of fluorescent labels, and by autoradiography in the case of an isotope label. Detection of the presence of the biological cell - and preferably also the amount of the cell - by visual inspection of the sample is preferred.

In a particularly preferred embodiment, the visual detection is based on a cut-off point above which one colour indicates the presence of the biological cell above a certain minimum amount (cut-off point), and below which cut-off point another colour indicates that the biological cell is present in an amount of less than that indicated by the cut-off point. When fluorescent markers are used the amounts of biological cell detected is directly correlatable with the fluorescence measured by a detection unit.

Enzyme-Linked Immuno-Sorbent Assays (ELISA) in which the biological cell is detected directly, initially by detection by a targeting species in the form of an antigen, hapten or antibody, and subsequently by means of an enzyme which is linked such as covalently coupled or conjugated either - when an antigen or hapten is to be determined - to an antibody which is specific for the antigen or hapten in question, or - when an antibody is to be determined - to an antibody which is specific for the antibody in question - are particularly preferred for detecting the predetermined biological cell according to the present invention.

In one preferred embodiment, the predetermined biological cell to be detected is bound or immobilized by immunochemically contacting the cell with a so-called "catching" antibody attached by e.g. non-covalent adsorption to the surface of an appropriate material such as a solid support. Examples of such solid support materials are polymers such as e.g. nitrocellulose or polystyrene, optionally in the form of a stick, a test strip, a bead or a microtiter tray.

Commercially available nitrocellulose membranes are for example
Millipore Hi-Flow Plus HF07504
Millipore Hi-Flow Plus HF09004
Millipore Hi-Flow Plus HF12004
Millipore Hi-Flow Plus HF13504
Millipore Hi-Flow Plus HF18004
Sartorius Unisart CN40
Sartorius Unisart CN90
Sartorius Unisart CN200 *

A suitable enzyme-linked specific antibody is allowed to bind to the immobilized biological cell to be directly detected. The amount of bound specific antibody, i.e. a parameter that is correlatable to the immobilized cell, is determined by adding a substance capable of acting as a substrate for the linked enzyme. Enzymatic catalysis of the substrate results in the development of a detectable signal such as e.g. a characteristic colour or a source of electromagnetic radiation. The intensity of the emitted radiation can be measured e.g. by spectrophotometry, by colorimetry, or by comparimetry. The determined intensity of the emitted radiation is correlatable - and preferably proportional - to the quantity of the predetermined biological cell to be detected. Examples of preferred enzymes for use in assays of this type are e.g. peroxidases such as horseradish peroxidase, alkaline phosphatase, glucose oxidases, galactosidases and ureases.

Immunochemical assays of a type analogous to ELISA, but employing other means of detection, are also suitable for detecting directly a biological cell according to the present invention. Such assays are typically based on the use of specific antibodies to which fluorescent or luminescent labelling species are covalently attached. So-called "time-resolved fluorescence" assays are particularly preferred and typically employ an europium ion label or an europium chelator, even though certain other lanthanide species or lanthanide chelators may also be employed. In contrast to many traditional fluorescent labelling species the fluorescence lifetime of lanthanide chelates is generally in the range of 100-1000 microseconds. In comparison, fluorescein has a fluorescence lifetime of only about 100 nanoseconds or less. By making use of a pulsed light source and a time-gated fluorometer, the fluorescence of lanthanide chelate compounds can be measured in a time-window of about 200-600 microseconds after each excitation. A main advantage of this technique is the reduction of background signals which may arise from more short-lived fluorescence of other substances present in the analysis sample or in the measurement system.

Additional assays employing immunochemical detection techniques capable of being exploited in the present invention belong to the group of "immunoblotting" procedures, such as e.g. "dot blot" and "western blot" procedures. In the western blot procedure, which is typically employed for the analysis and identification of antigenic polypeptides or proteins, the predetermined biological cell of interest is preferably transferred or fixed to a solid support or a membrane sheet such as e.g. a sheet of nitrocellulose or chemically treated paper to which the biological cell is capable of binding. Binding may be mediated by a targeting species such as e.g. an antibody bound to the support. An appropriate targeting species in the form of a specific antibody is initially added and later followed by a labelled second antibody against the first antibody. Labelled protein-A may be added as an alternative to the addition of labelled second antibody. The label is preferably a radioisotope, a fluorescent dye, an enzyme or a heavy metal such as gold or a colloid thereof. The presence and location of the biological cell is detected in an appropriate manner as described herein above.

"Connecting moiety" as used herein denotes any chemical species capable of forming a conjugate by binding a molecular species and a polymeric carrier molecule. The establishment, on the polymeric carrier molecule, of covalently bound reactive moieties deriving from divinyl sulfone, and the establishment of covalent bonds between, on the one hand, such moieties, and, on the other hand, molecular species as defined herein, are particularly preferred according to one embodiment of the invention.

Additional examples of connecting moieties, or reactive, functional groups, are chemical species comprising as a reactive group compounds such as e.g. 4-fluoro-3-nitrophenyl azide, acyl azides such as benzoyl azide and p-methylbenzoyl azide, azido formates such as ethyl azidoformate, phenyl azidoformate, sulfonyl azides such as benzenesulfonyl azide, phosphoryl azides such as diphenyl phosphoryl azide and diethyl phosphoryl azide, diazo compounds such as diazoacetophenone and 1-trifluoromethyl-1-diazo-2-pentanone, diazoacetates such as t-butyl diazoacetate and phenyl diazoacetate, beta-keto-alpha-diazoacetates such as t-butyl alpha diazoacetoacetate, aliphatic azo compounds such as azobisisobutyronitrile, diazirines such as 3-trifluoromethyl-3-phenyldiazirine, ketenes (-CH=C=O) such as ketene and diphenylketene, photoactivatable ketones such as benzophenone and acetophenone, peroxy compounds such as di-t-butyl peroxide, dicyclohexyl peroxide, diacyl peroxides such as dibenzoyl peroxide and diacetyl peroxide, and peroxyesters such as ethyl peroxybenzoate.

In the case of a vinyl group being the reactive, functional group, the reactivity of the vinyl group in a chemical species, such as e.g. divinyl sulfone, will generally require that the reactive functionality on the polymeric carrier, i.e. the group with which a vinyl group of e.g. a divinyl sulfone will react to form a covalent bond, is a nucleophilic function.

Suitable polymeric carriers will then be, for example, polymeric carriers with functional groups such as:
i) O⁻ (e.g. deprotonated phenolic hydroxy groups, such as deprotonated aromatic hydroxy groups in tyrosine residues of polypeptides or proteins),
ii) S⁻ (e.g. deprotonated thiol groups on aromatic rings or aliphatic groups, such as deprotonated thiol groups in cysteine residues of polypeptides or proteins),
iii) OH (e.g. aliphatic hydroxy groups on sugar rings, such as glucose or other monosaccharide rings in oligo- or polysaccharides; or alcoholic hydroxy groups in polyols, such as polyethylene glycols; or hydroxy groups in certain amino acid residues of polypeptides or proteins, such as serine or threonine residues),
iv) SH (e.g. thiol groups in cysteine residues of polypeptides or proteins), primary amino groups (e.g. in lysine or ornithine residues of polypeptides or proteins; or in amino-substituted sugar rings in certain polysaccharides or derivatives thereof, such as chitosan) or secondary amino groups (e.g. in histidine residues of polypeptides or proteins).

Accordingly, the functional group in question on molecular species in the context of the invention will also normally be a nucleophilic function, such as a nucleophilic function of one of the above-described types.

In one embodiment the present invention relates to a kit comprising a conjugate comprising a polymeric carrier molecule to which one or more molecular species are covalently attached, each via a connecting moiety in the form of a linking group.

One type of preferred linking groups are derived from divinyl sulfone. In this case the attachment of each of the linking groups to the polymeric carrier molecule is generated by a covalent linkage formed between one of the two vinyl groups of a divinyl sulfone molecule and a reactive functionality on the carrier molecule, and the attachment of a molecular species to the linking group being via a covalent linkage formed between the other vinyl group originating from the divinyl sulfone molecule and a functional group on the molecular species.

In particularly interesting conjugates of the latter type according to the invention, the polymeric carrier molecule further has covalently attached thereto one or more moieties derived from divinyl sulfone, each of which moieties is attached via a covalent linkage formed between one of the two vinyl groups of a divinyl sulfone molecule and a reactive functionality on the polymeric carrier molecule, at least one such said moiety in its attached state having the remaining vinyl group free and capable of reaction with a further molecular species having a functional group which is reactive towards the free vinyl group.

The molecular species attached to a conjugate according to the invention may be divided up into, for example, molecular species having molecular weights of about 2,000 or below, and molecular species having molecular weights of about 2,000 or above. In the former case, the polymeric carrier molecule of the conjugate may have from 1 to about 10,000 molecular species covalently attached thereto, for example from about 10 to about 1000 molecular species, such as from about 20 to about 500 molecular species covalently attached thereto. In the latter case, i.e. for molecular species of molecular weight about 2,000 or above, the polymeric carrier molecule of the conjugate may have from 1 to about 1000 molecular species covalently attached thereto, for example from 1 to about 500 molecular species, such as from 1 to about 100, from 2 to about 50, or from about 10 to about 50 molecular species covalently attached thereto.

"Polymeric carrier molecule" according to the invention is any polymer capable of binding a molecular species, or capable of modification with the purpose of binding a molecular species. Polymeric carrier molecules and conjugates comprising such polymeric carrier molecules according to the invention may be chosen from a wide variety of polymers, including:
i) natural and synthetic polysaccharides, as well as derivatives thereof, for example dextrans and dextran derivatives, starches and starch derivatives, cellulose derivatives, amylose and pectin, as well as certain natural gums and derivatives thereof, such as gum arabic and salts of alginic acid;
ii) homopoly(amino acid)s having suitable reactive functionalities, such as polylysines, polyhistidines or polyornithines;
iii) natural and synthetic polypeptides and proteins, such as bovine albumin and other mammalian albumins; and
iv) synthetic polymers having nucleophilic functional groups, such as polyvinyl alcohols, polyallyl alcohol, polyethylene glycols and substituted polyacrylates.

One group of preferred polymeric carrier molecules for the purposes of the invention are polysaccharides and derivatives thereof, for example: dextrans, carboxy-methyl-dextrans, hydroxyethyl- and hydroxypropyl-starches, glycogen, agarose derivatives, and hydroxyethyl- and hydroxypropyl-celluloses.

Dextrans have proved to be a group of particularly suitable polymers in connection with the present invention, and dextrans are presently representing one group of most preferred polymers.

The conjugates according to the present invention preferably have no net charge, since the presence of a net positive or negative charge may lead, inter alia, to undesirable non-specific binding of the conjugates to substances and/or materials other than those of interest. In many cases this condition will, unless charged molecular species are introduced, be fulfilled simply by ensuring that the polymeric carrier itself possesses no net charge. In a further embodiment of the invention, the polymeric carrier molecule of a reagent or conjugate of the invention is, in its free state, substantially linear and substantially uncharged at a pH in the range of about 4 to about 10. This pH interval is of practical relevance for the vast majority of immunochemical procedures, hybridization procedures and other applications of, notably, conjugates of the invention. Among various polymers which meet this criterion, are, for example, numerous polysaccharides and polysaccharide derivatives, e.g. dextrans and hydroxyethyl- and hydroxypropylcelluloses.

Depending on the use to which a reagent or conjugate of the invention is to be put, conjugates of the invention may be based on polymeric carrier molecules having molecular weights ranging from rather low to very high. In a further embodiment of the invention the polymeric carrier molecule may have a peak molecular weight in the range of from about 1,000 to about 40,000,000.

Peak molecular weights which are of considerable interest are in the range of about 1,000 to about 80,000, and in the range of about 80,000 to about 2,000,000. A peak molecular weight of particular interest, notably in the case of dextrans as polymeric carriers, is a peak molecular weight of about 500,000.

The term "peak molecular weight" (also denoted "peak average molecular weight") as employed herein denotes the molecular weight of greatest abundance, i.e. that molecular weight (among a distribution of molecular weights) which is possessed by the greatest number of molecules in a given sample or batch of the polymer. It is quite normal to characterize numerous types of polymers in this manner, owing to the difficulty (particularly for the highest molecular weights) of obtaining or preparing polymer fractions of very narrow molecular weight distribution. In the case of numerous commercially available polymers which are of interest in the context of the invention, for example dextrans, the manufacturer or distributor will be able to provide reliable peak molecular weight data (determined, for example, by gel-permeation chromatography) which can provide a basis for the selection of a polymer fraction suitable for the preparation of a particular type of reagent or conjugate.

Peak molecular weight values cited herein refer to the peak molecular weight of the free polymer in question, and take no account of, for example, the possible formation of cross-linked polymer units, e.g. as a result of cross-linking of two or more polymer molecules by reaction with e.g. divinyl sulfone during a process according to the invention for the preparation of a reagent or conjugate of the invention; such cross-linked units will, on average, have higher molecular weights than the individual free polymer molecules from which they are formed.

Conjugates according to the present invention may be tailored to meet a very wide range of requirements with regard to peak molecular weight of the polymer and the content of free, reactive vinyl groups. A further aspect of the invention relates to conjugates comprising a polymeric carrier molecule having a peak molecular weight of about 500,000 or about 2,000,000, or having a peak molecular weight in any one of the following ranges: From about 1,000 to about 20,000; from about 20,000 to about 80,000; from about 80,000 to about 500,000; from about 500,000 to about 5,000,000; and from about 5,000,000 to about 40,000,000;

The polymeric carrier molecules preferably have a content of free, reactive vinyl groups in the range of about 1 to about 5,000 µmoles of vinyl groups per gram of polymeric carrier, such as in any of the following sub-ranges (expressed in µmoles of vinyl groups per gram of polymeric carrier): From about 1 to about 50; from about 50 to about 300; from about 300 to about 1,000; and from about 1,000 to about 5,000.

In one further embodiment of the present invention there is provided a kit comprising a conjugate comprising a polymeric carrier having
i) a peak molecular weight of about 500,000 or about 2,000,000, or having a peak molecular weight in any one of the following ranges: From about 1,000 to about 20,000; about 20,000 to about 80,000; about 80,000 to about 500,000; about 500,000 to about 5,000,000; or about 5,000,000 to about 40,000,000; and
ii) a total content of molecular species and, where relevant, free vinyl groups in the range of about 1 to about 5,000 µmoles of a) molecular species and, where relevant, b) vinyl groups per gram of polymeric carrier, such as in any of the following sub-ranges (expressed in µmoles of molecular species plus, where relevant, µmoles of vinyl groups per gram of polymeric carrier): From about 1 to about 50; from about 50 to about 300; from about 300 to about 1,000; or from about 1,000 to about 5,000.

The samples according to the present invention are suitably conjunctival fluid, nasal secretion, pharyngeal secretion, sputum, mouth wash, bronchial wash, cervical and vaginal secretion, urine, blood, faeces, synovia, cerebrospinal fluid, ascites, vesicles, lesion exudate, and swabs from for example ulcers or conjunctiva, as well as waste water and drinking water as well as suspensions of water from for example ventilation ducts.

A wide variety of biological cells are capable of being directly detected by a targeting species according to the present invention. The biological cells are preferably detected by analysing a suitable body fluid sample as described herein above. The biological cell may be a mammalian cell, a microbial cell including a fungus, a yeast, and a bacterial cell, a virus particle including, but not limited to viral species belonging to the genus paramyxoviridae, such as e.g. a respiratory syncytial virus. Additional examples of biological cells including microbial cells including vira and bacteria capable of being directly detected are disclosed herein immediately below.

The following list includes viral species capable of being directly detected according to the invention

### Herpesvirus

Herpes Simplex Virus (HSV), Varicella Zoster Virus (VZV), Cytomegalovirus (CMV), Epstein-Barr Virus (EBV), and Human Herpes Virus-6 (HHV-6)

### Retrovirus

HIV-1, HIV-2, HTLV-I, and HTLV-11

### Hepatitisvirus

Hepatitis A Virus (HAV), Hepatitis B Virus (HBV), Hepatitis C Virus (HCV), Hepatitis D Virus (HDV), and Hepatitis E Virus (HEV)

### Rubellavirus

### Parvovirus B-19

### Morbillivirus

### Parotitisvirus

### Respirationsvirus

Influenza A and B Virus, Parainfluenza type 1, 2 and 3, Paramyxoviridae, Respiratory Syncytial Virus (RSV), Adenovirus.

### Enterovirus

Polio Virus, Coxsackie A Virus, Coxsackie B Virus, and Echovirus

### Cardiovirus

Encephalomyocarditis virus and meningovirus,

### Aphthoviruses

Foot-and-mouth disease virus serotype A, O, C, SAT1, SAT2, SAT3 or Asia1.

### Gastroenteritisvirus

Rotavirus, Adenovirus, Norwalkvirus, and enteropatogic vira in general (SRV-komplex)

### Human Papillomavirus (HPV)

### Exotic vira

Denguevirus, Yellow Fever Virus, and other Arbovira

Additional viral species capable of being directly detected according to the invention are: Poxvirus, Papovavirus, Parvovirus, Picornavirus, Togavirus, Myxovirus, Paramyxovirus, Reovirus, Rhabdovirus, Retrovirus, and Arenavirus.

The following list includes bacteria capable of being directly detected according to the invention.

### Aerobic and facultative aerobic Gram-positive bacteria

### Micrococcaceae

Staphylococcus aureus, Staphylococcus epidermidis, and Staphylococcus saprophyticus

### Streptococcus and enterococcus

Streptoccocus pyogenes (Lancefield grp. A), Streptococcus agalactiae (Lancefield grp. B), Streptococcus (Lancefield grp. C, F, G), Enterococcus faecalis and other Enterococci (e.g. Lancefield grp D), Streptococcus pneumoniae.

### Lactobacillus

### Listeria

### Corynebacterium

Corynebacterium diphteriae

### Propionibacterium

Propionibacterium acnes

### Actinomyces

### Norcardia

### Acid resistant bacteria

### Mycobacteriaceae

Mycobacterium tuberculosis, Mycobacterium leprae

### Gram-positive, endosporeforming bacteria

### Bacillus

Bacillus anthracis, Bacillus cereus

### Clostridum

Clostridium tetani, Clostridium botulinum, Clostridium difficile, Clostridium perfringens

### Anaerobic Gram-positive and Gram-negative cocci

### Peptostreptococcus

### Anaerobic Gram-negative bacteria

### Bacteriodes

Bacteriodes fragilis

### Aerobic Gram-negative diplococci and coccibacillae

Neisseria, Neisseria meningitidis, Neisseria gonorrhoeae

### Gram-negative rodforming bacteria

### Bordetella

Bordetella pertussis, Bordetella parapertussis

### Brucella

Brucella melitensis, Brucella abortus, Brucella suis

### Haemophilus

Haemophilus influenzae, Haemophilus ducreyi

### Pasteurella

Pastereulla multocida

### Gardnerella

Gardnerella vaginalis

### Capnocytophaga

Capnocytophaga canimorsus

### Non-fermentative Gram-negative bacteria

### Acinebacter and Flavobacterium

### Pseudomonas

Pseudomonas aeruginosa

### Aerobic Gram-negative bacteria

### Legionella

Legionella pneumophila

### Facultative anaerobic Gram-negative rods

### Enterobacteriaceae

### Escherichia

Escherichia coli

### Shigella

Shigella dysenteriae, Shigella flexneri, Shigella boydii, Shigella sonnei

### Salmonella

Salmonella typhi, Salmonella paratyphi (A, B, C), Salmonella typhimurium, Salmonella enterididis

### Yersenia

Yersenia pestis, Yersenia enterocolotica

### Vibrio

Vibrio cholerae (klassisk, El Tor)

### Curved and helical-shaped bacteria

### Campylobacter

Campylobacter jejuni

### Helicobacter

Helicobacter Pylori

### Treponema

Treponema pallidum

### Borrelia

Borrelia burgdorferi, Borrelia recurrentis

### Leptospiraceae

Leptospiraceae interrogans

### Chlamydiales

Chlamydia trachomatis, Chlamydia psittaci, Chlamydia pneumoniae

### Rickettsiales

Rickettsia

### Coxiella

Coxiella burnetii

### Mycoplasmatales

### Mycoplasma

Mycoplasma pneumoniae

### Ureaplasma

Ureaplasma urealyticum

Additional examples of microbial cells capable of being directly detected according to the present invention are: Achromobacter xylosoxidans, Acinetobacter calcoaceticus, preferably A. anitratus, A. haemolyticus, A. alcaligenes, and A. lwoffii, Actinomyces israelii, Aeromonas hydrophilia, Alcaligenes species, preferably A. faecalis, A. odorans and A. denitrificans, Arizona hinshawii, Bacillus anthracis, Bacillus cereus, Bacteroides fragilis, Bacteroides melaninogenicus, Bordetella pertussis, Borrelia recurrentis, Brucella species, preferably B. abortus, B. suis, B. melitensis and B. canis, Calymmatobacterium granulomatis, Campylobacter fetus ssp. intestinalis, Campylobacter fetus ssp. jejuni, Chlamydia species, preferably C. psittaci and C. trachomatis, Chromobacterium violaceum, Citrobacter species, preferably C. freundii and C. diversus, Clostridium botulinum, Clostridium perfringens, Clostridium difficile, Clostridium tetani, Corynebacterium diphtheriae, Corynebacterium, preferably C. ulcerans, C. haemolyticum and C. pseudo-tuberculosis, Coxiella burnetii, Edwardsiella tarda, Eikenella corrodens, Enterobacter, preferably E. cloacae, E. aerogenes, E. hafniae (also named Hafnia alvei) and E. agglomerans, Erysipelothrix rhusiopathiae, Escherichia coli, Flavobacterium meningosepticum, Francisella tularensis, Fusobacterium nucleatum, Gardnerella vaginalis, Haemophilus ducreyi, Haemophilus influenzae, Helicobacter species, Klebsiella species, preferably K. pneumoniae, K. ozaenae and K. rhinoscleromatis, Legionella species, Leptospira interrogans, Listeria mono-cytogenes, Moraxella species, preferably M. lacunata and M. osloensis, Mycobacterioum bovis, Mycobacterium leprae, Mycobacterium tuberculosis, Mycoplasma species, preferably M. pneumoniae, Neisseria gonorrhoeae, Nelsseria meningitidis, Nocardia species, preferably N. asteroides and N. brasiliensis, Pasteurella multocida, Peptococcus magnus, Plesiomonas shigelloides. Proteus species, preferably P. mirabilis, P. vulgaris, P. rettgeri and P. morganli (also named Providencla rettgeri and Morganella morganil respectively), Providencia species, preferably P. alcalifaciens, P. stuartii and P. rettgeri (also named Proteus rettgeri), Pseudomonas aeruginosa, Pseudomonas mallei, Pseudomonas pseudomallel, Rickettsia, Salmonella species, preferably S. enteridis, S. typhi and S. derby. Serratia species, preferably S. marcescens, Shigella dysenteriae, S. flexneri, S. boydii and S. sonnei, Spirillum minor. Staphylococcus aureus, Staphylococcus epidermidis, Staphylococcus saprophyticus, Streptobacillus moniliformis, Streptococcus, preferably S. faecalis, S. faecium and S. durans, Streptococcus agalactiae, Streptococcus pneumoniae, Streptococcus pyogenes, Treponema carateum, Treponeam pallidum, Treponema pertenue, preferably T. pallidum, Ureaplasma urealyticum, Vibrio cholerae, Vibrio parahaemolyticus, Yersinia enterocolitica, and Yersinia pestis.

Further examples of lower eucaryotes Including yeast and fungi capable of being detected directly are: Phycomycetes, ascomycets, basidiomycets, deuteromycetes and fungi imperfecti.

The method of diagnosis may thus be able to identify individuals suffering from a condition selected from the group consisting of, but not limited to: Actinomycosis, Adenovirus-infections, Antrax, Bacterial dysentery, Botulisme. Brucellosis (Bang's disease), caused by e.g. B. melitensis and B. suis, Candidiasis, Cellulitis, Chancroid. Cholera, Coccidioidomycosis, acute afebrile Conjunctivitis, Cystitis, Dermatophytosis, Bacteriel Endocarditis, Epiglottitis, Erysipelas, Erysipeloid, Gastroenteritis, Genital herpes, Glandulae, Gonorrhea. Viral Hepatitis. Histoplasmose. Impetigo, Malaria, Mononucleosis, Influenza, Legionaires disease, Leptospirosis, Lyme disease, Melioidosis, Meningitis, Nocardiosis Nocardia asteroides, Non-gonococcal urethritis, Pinta, Pneumococcal lung disease, Poliomyelitis, Primary lung infection, Pseudomembranious enterocolitis, antibiotic-associated Puerperal sepsis, Rabies, Relaps-fever, Rheumatic fever, Rocky Mountain spotted-fever, Rubella, Rubeola, Staphylococcal scalded skin syndrome, Streptococcal pharyngitis (strep throat), Syphilis, Tetanus, Toxic shock syndrome, Toxoplasmose, Tuberculosis, Tularemia, Typhoid fever, Typhus, Vaginitis, Varicella, Verrucae, Pertussis, Framboesia (Yaws) and Yellow fever.

In addition to detection of the biological cell it may be convenient and advantageous to determine the presence and/or amount of one or more inflammatory indicators, for example in order to i) assess severity, or ii) predict prognosis and/or severity of the infection.

Inflammatory indicators including inflammatory mediators according to the invention are selected from, but not limited to, the group consisting of cytokines and autoantibodies, including cytokines belonging to inflammatoric systems such as e.g. the IL-1 system; including IL-1α and IL-1β, IL-1ra, and autoantibodies against IL-1α, sIL1-RI and sIL1-RII, the TNFα system; including, but not limited to TNFα antagonists sTNFR p55 and p75, and IL-6 and autoantibodies against IL-6, cytokines belonging to immunoregulatory systems such as mediators related to the Th1/Th2 balance: IL-12, sIL-4R, Th1 cytokines: TNFβ (LT), INFγ, Th2 cytokines: IL-4, IL-10, and additional mediators such as e.g. IL-2, RANTES, IL-8, sIL-2R, IL-18, IFNα and eosinofil cationic protein.

The below listed cytokines represent one preferred group of inflammatory indicators according to the present invention.

| **Cytokine** | **Produced by for example** | **Most important effects** |
|---|---|---|
| IL-1α/ IL-1β | Monocytes, macrophages, NK-cells, T- and B-cells, neutrophil granolocytes, keratinocytes, endothelial cells, astrocytes, fibroblasts, synovial cells, smooth muscle cells, mesangial cells | Activates T-, B- and NK-cells, endothelial cells, osteoclasts and bone marrow cells. Numerous effects on inflammatory cells, mediates fever |
| IL-1ra | Monocytes, macrophages | Antagonizes effects of IL-1α/β on receptor level |
| IcIL-1ra | Neutrophil granulocytes, keratinocytes | Is being utilised on a trial basis in patients with sepsis, chronic arthritis |
| IL-2 | Th1-cells | Enhances growth of T-, B- and NK-cells |
| IL-3 | Th2-cells, keratinocytes, mastcells | Stimulates basophilic granulocytes |
| IL-4 | Th2-cells, basophilic granulocytes and mastcells, monocytes/macrophages, B-cells | Enhances growth of T- and B-cells. Suppresses macrophage functions. Induces shift of isotype in B-cells (IgE- and IgG4-production) |
| IL-5 | Th2-cells, mastcells | Stimulates eosinophil granulocytes |
| IL-6 | Monocytes, macrophages, Th2-cells, | As IL-1 with certain exceptions. |
| | fibroblasts, endothelial cells, certain cancer cells | Most important activator of acut-phase protein production from hepatocytes. |
| | | Growth factor for myeloma cells |
| IL-7 | Stroma cells in bone marrow, foetal liver cells, cells in intestinal epithelium | Growth- and maturation of pre-T and pre-B-cells. Coactivates T- and NK-cells |
| IL-8 | α-chemokine | α-chemokine |
| IL-9 | Th2-cells | Growth factor for mastcells (with IL-3), T-cells, megakaryocytes, pre-erythrocytes (with erythropoietin). Coactivates mast-cells, T-cells and B-cells (IgE-production) |
| IL-10 | Monocytes, macrophages, Th0-, Th2-cells, B-cells (especially EBVⁱ in- | Coactivates certain T- and B-cell subpopulations and mastcells. |
| | fected), mastcells, keratinocytes, epidermal cells | Suppresses Th1-cells (IFNγ-production) and certain monocyte/ macrophage- and NK-cell functions |
| IL-11 | Fibroblasts, stroma cells in bone marrow, foetal lung cells, trophoblasts | As IL-6 |
| IL-12 | Monocytes, macrophages, B-cells, dendritic cells, Langerhan cells, keratinocytes, neutrophil granulocytes | Activates Th1-cells (IFNγ-production) and NK-cells |
| IL-13 | T-cells | Stimulates B-cell growth (induces IgE, IgG4) |
| IL-14 | T-cells | Coactivates B-cells (proliferation/differentiation), but inhibits Ig-secretion |
| IL-15 | Monocytes, macrophages, certain T-cells, fibroblasts, endothelial and epithelial cells, myocytes, stroma cells in bone marrow, placenta cells | As IL-2 |
| IL-16 | T-cells, eosinophil granulocytes | Chemotactic for CD4 T-cells. Activates CD4 T-cells and monocytes |
| IL-17 | Th-cells | Activates T-cells, fibroblasts (ICAM-1 expression). Induces IL-6 and IL-8 |
| IL-18 | Monocytes, macrophages, Kupffer cells, osteoblasts | Activates Th1- and NK-cells (induces IFNγ) |
| LIF | Monocytes, macrophages, T-cells, | As IL-6 |
| | stroma cells in bone marrow, fibroblasts, astrocytes | |
| PDGF | Thrombocytes, monocytes, macrophages, endothelial cells, smooth muscle cells, fibroblasts, neurons | Activates vascular smooth muscle cells, endothelial- and epithelial cells, gliacells, chondrocytes, fibroblasts, neutrophilocytes and monocytes. |
| | | Chemotactic for the above mentioned cells |
| NGF | Macrophages, astrocytes, nerve cells, smooth muscle cells, fibroblasts | Activates B-cells, basophilic granulocytes and sympathetic and sensory neurons |
| TGFβ (several forms) | Megakaryocytes, thrombocytes, monocytes, macrophages, T-cells, | Activates B-cells (induces IgA), osteoblasts, fibroblasts and other cells. |
| | endothelial cells, fibroblasts, osteoblasts, chondrocytes, smooth muscle cells | Inhibits growth of endothelial and epithelial cells, osteoclasts, T-cells and NK-cells |
| TNF | Monocytes, macrophages incl. tissue macrophages, Th1- and Tc-cells, B-cells, NK-cells, neutrophilocytes, ke- | Activates T-, B- and NK-cells, neutro- and eosinophil granulocytes, endothelial cells, osteoclasts. |
| | ratinocytes, smooth muscle cells | Cytotoxic for transformed and virus infected cells. Mediates fever |
| LTα/LTβ | Th1-cells, (B-cells) | As TNF |
| FasL | Th1- and Tc-cells, NK-cells | Cytotoxic for virus infected cells, incl. HIV-infected cells |
| IFNα (>16 subtypes) | Virus infected leukocytes, T- and B- | Antiviral activity. |
| | cells, monocytes, macrophages | Antiproliferative and antitumour effects. Activates macrophages, NK-cells and B-cells |
| IFNβ | Many virus infected cell types, fibroblasts | Antiviral activity. Activates NK-cells |
| IFNγ | Th-1- and Tc-cells, endothelial cells, smooth muscle cells | Activates fibroblasts, monocytes/ macrophages, T-, B- and NK-cells (induces IgG). Induces MHC II (many cell types). |
| | | Suppresses cell growth in general |
| SCF | Stroma cells in bone marrow, endothelial cells, fibroblasts, Sertoli cells | Activates and differentiates marrow stem-cells, mastcells |
| GM-CSF | Th2- cells, fibroblasts, endothelial cells, macrophages, mastcells, neutrophil granulocytes, eosinophil granulocytes | Activates, differentiates precursors of T-cells, monocytes, neutrophil granulocytes |
| G-CSF | Monocytes, macrophages, fibroblasts, endothelial cells, T-cells, neutrophil granulocytes | Activates, differentiates precursors of neutrophil granulocytes |
| M-CSF | Monocytes, macrophages, fibroblasts, endothelial cells | Activates, differentiates precursors of monocytes |
| α-chemokines (CXC) | Monocytes, macrophages, T-cells, endothelial cells, several other cells | Chemotactic for neutrophil granulocytes, T-cells, basophilic granulocytes, keratinocytes |
| β-chemokines (CC) | Monocytes, macrophages, T- and B-cells, thrombocytes, endothelial cells, smooth muscle cells, mastcells, fibro- | Chemotactic for monocytes/ macrophages, NK-cells, eosinophil and basophilic granulocytes. |
| | blasts | Inhibits infection of CD4-positive monocytes/ macrophages with HIV (that uses β-chemokine receptors as co-activators by the infection) |
| Lymfotactin | T-cells | Chemotactic for T-cells |
| VIP/PACAP and other neuropeptides | Nerve cells (in for example thymus, spleen and lymphnodes), T- and B- | Immunosuppressive by inhibition of IL-2 and IL-4 production. |
| | cells, eosinophilocytes, mastcells, neutrophilocytes | Indirect immunostimulation by inhibition of IL-10 |

### Abbreviations and definitions

- CC:: β-chemokines (for example macrophage inflammatory protein (MIP)-1, monocyte chemoattractant protein (MCP)-1-4, regulated on activation, normal T expressed and secreted (RANTES)
- CXC:: α-chemokines (for example IL-8)
- CSF:: colony-stimulating factor
- FasL:: Fas-ligand
- G-CSF:: granolocyte-CSF
- GM-CSF:: granolocyte-macrophage-CSF
- icIL-1ra:: intracellular IL-1ra
- IFN:: interferon
- IL:: interleukine
- LT:: lymphotoxin
- M-CSF:: macrophage-CSF
- MHC:: major histocompatibility complex
- NGF:: nerve growth factor
- NK-cells:: natural killer cells
- PACAP:: pituitary adenylyl cyclase-activating peptide
- SCF:: stem-cell factor
- TGF:: transforming growth factor
- TNF:: tumour necrosis factor
- VIP:: vasoactive intestinal peptide

In particular the present invention may be used for providing a cytokine profile, i.e. a measurement of at least two cytokines, such as for example at least four cytokines, whereby the presence and relative concentration of each cytokine may be indicative of a disease or of the prognosis of a disease.

In the following the invention is exemplified by using an RS virus as a biological cell.

### Example 1

Dipstick for detecting RS virus in a sample

A dipstick for detecting RS virus in a sample that could clearly detect RS virus, by the appearance of a clear visually detectable signal, such as a red spot in a functional lateral flow assay was developed.

The antigen to be tested is a commercially available antigen (Chemicon, RSV, long AG 857).

A monoclonal antibody directed to F-glycoprotein as the targeting species coupled to the solid surface on the dipstick, the so-called catching antibody was used.

The reporter species further comprised polydextran polymeric carrier molecules, which were of approximately 500.000 Da, to which the reactive group divinylsulphone were covalently attached. Furthermore, the reporter species comprised rhodamine label molecules, which were also attached via the divinylsulphone groups.

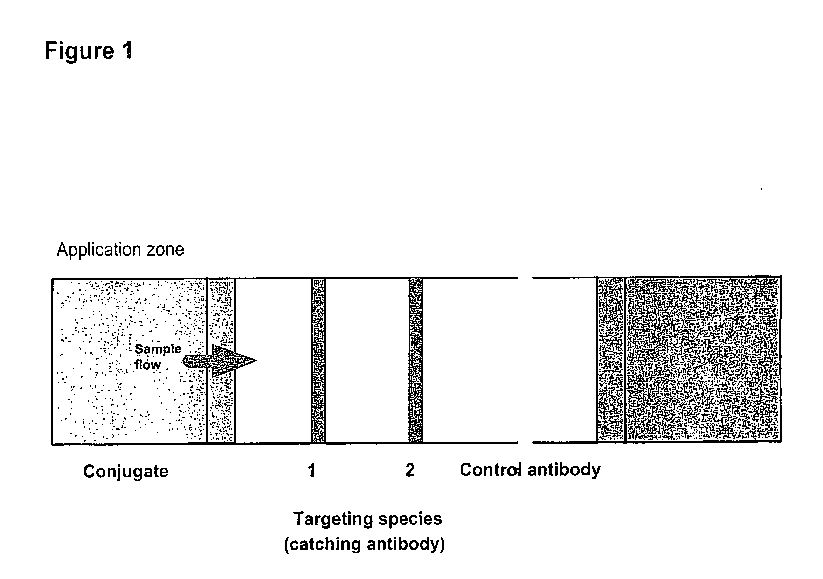

To test the reporter species a 2-layer lateral flow test was employed, following the principles outlined in figure 1. Figure 1 illustrates a schematic dipstick, for use in an assay for testing detecting of RS related cell in a sample. The dipstick comprises an application zone for the sample comprising the reporter species. The term conjugate refers to reporter species. Furthermore, the dipstick comprises one zone whereto the catching antibody is coupled and a second zone whereto the control antibody is coupled. The dipstick is made of nitrocellulose (Sartonius Unisart CN200), and of a microporous membrane of. Whatman FO 75-17 or Millipore Rapid Q24, and an absorbant pad (Whatman 1,5 NF).

A secondary antibody with specificity against the targeting antibody comprised within the reporter species was used as catching antibody. This lateral test gave a positive red spot, which showed that 1) targeting antibody was coupled to polydextran carrier; 2) the polydextran carrier had good flow characteristics. Furthermore, none of them gave rise to background/unspecific binding.

The test was developed so that a visually visible red spot appears when the test is positive. This spot is produced by accumulation of rhodamine linked to the reporter species. The positive result in the test is defined as samples comprising RS virus. A negative result, which is visualised by no colour change (no red spot appear), was obtained when substantially no RS virus was used.

The test is a 1-step test, where sample is applied directly to the dipstick after which the test results appear. When the test is performed as a 1-step test the first colour reaction appear on the flow test as early as after 1-3 minutes. The test is finished after about 5 minutes.

A control antibody that binds the reporter species independently of the antigen in the urine, was also coupled to the solid surface of the dipstick within the control zone. A red control spot appeared every time in the test regardless whether negative urine or positive urine was used, as an indicator of whether the test was correctly performed. The red colour of this control spot was also produced by accumulation of rhodamine linked to the reporter species.

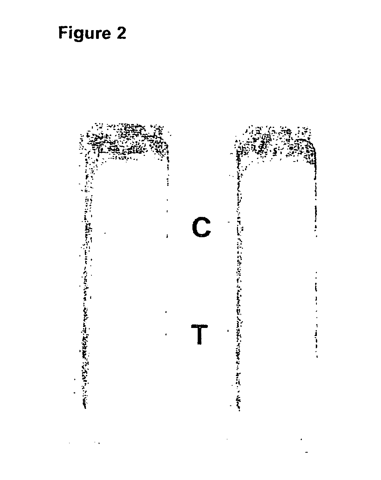

Furthermore, a dipstick has been developed so that a red test line appears across the membrane instead of a red spot, both for observing the test result and the control (figure 2). Often it is observed that the colour intensity is increased on a test line compared to a test spot.

### Example 2

### Competetive dispstick

In this example a dipstick similar to the dipstick described in Example 1 was produced as a competitive dipstick whereby a positive signal is shown as no change of colour, whereas a negative signal is shown as a colour change.

The amount of reporter species was titrated in a way such as a red spot (visible accumulation of rhodamine) only appeared in negative samples.

## Claims

1. A kit for directly detecting a predetermined biological cell present in a sample in an amount of less than about 2000 per microlitre (10⁻⁶ litre) sample, said kit comprising
an extended solid phase comprising
i) a solid support, and
ii) a plurality of a first targeting species bound to the solid support, said targeting species being capable of directly binding said predetermined biological cell when it is present in a sample that is brought into contact with the solid support, and
a mobile solid phase comprising
iii) a conjugate comprising
a polymeric carrier molecule bound to
a) at least one first and/or second targeting species capable of directly binding said predetermined biological cell when it is present in a sample that is brought into contact with the solid support, and
b) at least one labelling species, and
wherein the conjugate comprises
A) a polymeric carrier molecule comprising a plurality of at least one reactive, functional group.
B) at least one connecting moiety attached to the at least one reactive, functional group,
C) at least one molecular species selected from the group of molecular species consisting of targeting species and labelling species, wherein each of the molecular species comprises at least one functional group that is reactive with at least one connecting moiety attached to the reagent,
D) wherein the conjugate comprises at least one molecular species covalently attached thereto via a connecting moiety,
wherein said solid support is **characterized by**:
iv) an application zone for applying the sample comprising a biological cell, said zone comprising at least one conjugate as defined in iii) above, said conjugate being movable, and said application zone being in liquid contact with
v) a detection zone for detecting the presence, amount or concentration of said at least one conjugate, said zone further comprising the plurality of a first targeting species bound to the solid support.

2. The kit according to claim 1, wherein the kit is a micro flow device or a lateral flow device.

3. The kit according to any of claims 1-3, wherein the polymeric carrier molecule comprises reactive, functional groups in an amount of from about 5 to about 5,000 µmoles per gram of polymeric carrier.

4. The kit according to any of claims 1 and 2, wherein the biological cell capable of being directly detected by a targeting species is a mammalian cell.

5. The kit according to any of claims 1 and 2, wherein the biological cell capable of being directly detected by a targeting species is a microbial cell.

6. The kit according to any of claims 4 and 5, wherein the biological cell capable of being directly detected by a targeting species is a virus particle.

7. The kit according to claim 6, wherein the virus capable of being directly detected by a targeting species belongs to the genus paramyxoviridae.

8. The kit according to claim 7, wherein the virus is respiratory syncytial virus.

9. The kit according to claim 8, wherein the virus capable of being directly detected by a targeting species belongs to the family of picorna viruses.

10. The kit according to claim 9, wherein the picorna virus capable of being directly detected by a targeting species is a foot-and-mouth disease virus.

11. The kit according to claim 5, wherein the microbial cell is selected from the group consisting of a fungus, a yeast, and a bacterial cell.

12. The kit according to any of claims 1 and 2, wherein the targeting species is selected from the group of species consisting of antigens; haptens; monoclonal and polyclonal antibodies; gene probes; natural and synthetic oligo- and polynucleotides; natural and synthetic mono-, oligo- and polysaccharides; lectins; avidin and streptavidin; biotin; growth factors; hormones; receptor molecules; protein A; and protein G.

13. The kit according to any of claims 1 and 2, wherein the labelling species is selected from the group of species consisting of proteins; enzymes; toxins; drugs; dyes; fluorescent, luminescent, phosphorescent and other light-emitting substances; metal-chelating substances; substances labelled with a radioactive isotope; and substances labelled with a heavy atom.

14. The kit according to any of claims 1 and 2, wherein the labelling species is selected from the group of species consisting of ferritin, phycoerythrins, phycocyanins, phycobilins, horseradish peroxidase, alkaline phosphatase, glucose oxidases, galactosidases, ureases, iminodiacetic acid, ethylenediaminetetraacetic acid, diethylenetriaminepentaacetic acid, and desferrioxamine B.

15. The kit according to any of claims 1 and 2, wherein the first and second targeting species are identical.

16. The kit according to any of claims 1 and 2, wherein the first and second targeting species are non-identical.

17. The kit according to any of claims 1 and 2, wherein the polymeric carrier is selected from the group of polymers consisting of natural and synthetic polysaccharides; homopoly amino acids; natural and synthetic polypeptides and proteins; and synthetic polymers having nucleophilic functional groups.

18. The kit according to any of claims 1 and 2, wherein the polymeric carrier is selected from the group of polymers consisting of polyvinyl alcohols, polyallyl alcohols, polyethylene glycols and substituted polyacrylates.

19. The kit according to any of claims 1 and 2, wherein the polymeric carrier is selected from the group consisting of dextrans, carboxymethyl-dextrans, starches, hydroxyethyl-starches, hydroxypropyl-starches, glycogen, agarose derivatives, cellulose derivatives and natural gums.

20. The kit according to claim 7, wherein the polymeric carrier is a dextran.

21. The kit according to any of claims 1 and 2, wherein the polymeric carrier is selected from the group consisting of hydroxyethyl-celluloses and hydroxypropyl-celluloses.

22. The kit according to any of the preceding claims, said kit being in form of a dipstick.

23. The kit according to any of the preceding claims 1-22, said kit being adapted for a microsystem.

24. A method of detecting a predetermined biological cell present in a sample, said method comprising the steps of
i) contacting the sample with the kit of any of claims 1 to 19, and
ii) detecting a targeting species capable of targeting the predetermined biological cell,
wherein the detection of the targeting species is indicative of the presence of the biological cell in the sample.

25. The method according to claim 24, wherein the sample is a body fluid sample.

26. The method according to claim 24 or 25, for further detecting a predetermined inflammatory indicator present in a sample in an amount of less than about 2000 per microlitre (10⁻⁶ litre), said method comprising the steps of
i) contacting the sample with a kit comprising
a) a solid support, and
b) a plurality of a first targeting species bound to the solid support, said targeting species being capable of directly binding said predetermined inflammatory indicator when it is present in a sample that is brought into contact with the solid support, and
c) a conjugate comprising a polymeric carrier molecule bound to i) at least one first and/or second targeting species capable of directly binding said predetermined inflammatory indicator when it is present in a sample that is brought into contact with the solid support, and ii) at least one labelling species,
and
ii) detecting a targeting species capable of targeting the predetermined inflammatory indicator,
wherein the detection of the targeting species is indicative of the presence of the predetermined inflammatory indicator in the sample.

27. The method according to claim 26, wherein the polymeric carrier molecule comprises i) a plurality of at least one reactive, functional group, ii) at least one connecting moiety attached to the at least one reactive, functional group, and iii) at least one molecular species selected from the group of molecular species consisting of targeting species and labelling species, wherein each of the molecular species comprises at least one functional group that is reactive with at least one connecting moiety attached to the reagent, and wherein the conjugate comprises at least one molecular species covalently attached thereto via a connecting moiety.

28. The method according to any of claims 24-27, wherein the targeting species is selected from the group of species consisting of antigens; haptens; monoclonal and polyclonal antibodies; gene probes; natural and synthetic oligo- and polynucleotides; natural and synthetic mono-, oligo- and polysaccharides; lectins; avidin and streptavidin; biotin; growth factors; hormones; receptor molecules; protein A; and protein G.

29. The method according to any of claims 24-28, wherein the labelling species is selected from the group of species consisting of proteins; enzymes; toxins; drugs; dyes; fluorescent, luminescent, phosphorescent and other light-emitting substances; metal-chelating substances; substances labelled with a radioactive isotope; and substances labelled with a heavy atom.

30. The method according to any of claims 24-29, wherein the labelling species is selected from the group of species consisting of ferritin, phycoerythrins, phycocyanins, phycobilins, horseradish peroxidase, alkaline phosphatase, glucose oxidases, galactosidases, ureases, iminodiacetic acid, ethylenediaminetetraacetic acid, diethylenetriaminepentaacetic acid, and desferrioxamine B.

31. The method according to any of claims 24-30, wherein the polymeric carrier is selected from the group of polymers consisting of natural and synthetic polysaccharides; homopoly amino acids; natural and synthetic polypeptides and proteins; and synthetic polymers having nucleophilic functional groups.

32. The method according to any of claims 24-30, wherein the polymeric carrier is selected from the group of polymers consisting of polyvinyl alcohols, polyallyl alcohols, polyethylene glycols and substituted polyacrylates.

33. The method according to any of claims 24-30, wherein the polymeric carrier is selected from the group consisting of dextrans, carboxymethyl-dextrans, starches, hydroxyethyl-starches, hydroxypropyl-starches, glycogen, agarose derivatives, cellulose derivatives and natural gums.

34. The method according to claim 32, wherein the polymeric carrier is a dextran.

35. The method according to any of claims 24-30, wherein the polymeric carrier is selected from the group consisting of hydroxyethyl-celluloses and hydroxypropyl-celluloses.

36. The method according to any of claims 26 to 35, wherein the predetermined inflammatory indicator is selected from the group consisting of agonists from the IL-1 system, preferably IL-1α, IL-1β, IL-1ra, autoantibodies against IL-1α, sIL1-RI and sIL1-RII.

37. The method according to any of claims 26 to 35, wherein the predetermined inflammatory indicator is selected from the group consisting of agonists from the TNFα system, preferably sTNFR p55 and p75.

38. The method according to any of claims 26 to 35, wherein the predetermined inflammatory indicator is selected from the group consisting of IL-6 and autoantibodies against IL-6.

39. The method according to any of claims 26 to 35, wherein the predetermined inflammatory indicator is selected from the group consisting of IL-12, sIL-4R, TNFβ (LT), INFγ, IL-4, and IL-10.

40. The method according to any of claims 26 to 35, wherein the predetermined inflammatory indicator is selected from the group consisting of IL-2, RANTES, IL-8, sIL-2R, IL-18, IFNα, and eosinophil cationic protein.

41. A method for diagnosing an infectious condition in an individual, said method comprising the steps of
i) detecting a predetermined biological cell present in a body fluid sample according to any of claims 24-40, and
ii) diagnosing said infectious condition.

42. The method of claim 41 said method comprising the further steps of
i) detecting a predetermined inflammatory indicator present in a body fluid sample according to any of claims 26 to 40, and
ii) diagnosing said infectious condition.

## Patentansprüche

1. Kit zum direkten Nachweisen einer vorbestimmten biologischen Zelle, die in einer Probe in einer Menge von weniger als ungefähr 2.000 pro Mikroliter (10⁻⁶ Liter) Probe anwesend ist, der Kit umfasst
eine erweiterte feste Phase, umfassend
i) einen festen Träger, und
ii) eine Vielzahl einer ersten Zielspezies, die an den festen Träger gebunden ist, die Zielspezies ist in der Lage, die vorbestimmte biologische Zelle direkt zu binden, wenn sie in einer Probe anwesend ist, die mit dem festen Träger in Kontakt gebracht wird, und
eine mobile feste Phase, umfassend
iii) ein Konjugat, umfassend
ein polymeres Trägermolekül, gebunden an
a) mindestens eine erste und/oder zweite Zielspezies, die in der Lage ist, die vorbestimmte biologische Zelle direkt zu binden, wenn sie in einer Probe anwesend ist, die mit dem festen Träger in Kontakt gebracht wird, und
b) mindestens eine Markierungsspezies, und
wobei das Konjugat umfasst
A) ein polymeres Trägermolekül, umfassend eine Vielzahl mindestens einer reaktiven funktionellen Gruppe,
B) mindestens einen Verbindungsrest, der an die mindestens eine reaktive funktionelle Gruppe angeheftet ist,
C) mindestens eine molekulare Spezies, die ausgewählt ist aus der Gruppe molekularer Spezies, bestehend aus Zielspezies und Markierungsspezies, wobei jede der molekularen Spezies mindestens eine funktionelle Gruppe umfasst, die mit mindestens einem Verbindungsrest, der an das Reagens angeheftet ist, reaktiv ist,
D) wobei das Konjugat mindestens eine molekulare Spezies umfasst, die kovalent über einen Verbindungsrest daran angeheftet ist, und
wobei der feste Träger **gekennzeichnet ist durch**:
iv) eine Auftragungszone zum Auftragen der Probe, umfassend eine biologische Zelle, die Zone umfasst mindestens ein Konjugat, wie in iii) oben definiert ist, das Konjugat ist beweglich, und die Auftragungszone befindet sich in flüssigem Kontakt mit
v) einer Nachweiszone zum Nachweisen der Anwesenheit, Menge oder Konzentration des mindestens einen Konjugats, die Zone umfasst weiterhin eine Vielzahl einer ersten Zielspezies, die an den festen Träger gebunden ist.

2. Kit gemäß Anspruch 1, wobei das Kit eine Mikroflussvorrichtung oder eine Lateralflussvorrichtung ist.

3. Kit gemäß einem der Ansprüche 1 - 3, wobei das polymere Trägermolekül reaktive funktionelle Gruppen in einer Menge von ungefähr 5 bis ungefähr 5.000 µMol pro Gramm des polymeren Trägers umfasst.

4. Kit gemäß einem der Ansprüche 1 und 2, wobei die biologische Zelle, die in der Lage ist, durch eine Zielspezies direkt nachgewiesen zu werden, eine Säugetierzelle ist.

5. Kit gemäß einem der Ansprüche 1 und 2, wobei die biologische Zelle, die in der Lage ist, durch eine Zielspezies direkt nachgewiesen zu werden, eine mikrobielle Zelle ist.

6. Kit gemäß einem der Ansprüche 4 und 5, wobei die biologische Zelle, die in der Lage ist, durch eine Zielspezies direkt nachgewiesen zu werden, eine Viruspartikel ist.

7. Kit gemäß Anspruch 6, wobei das Virus, das in der Lage ist, durch eine Zielspezies direkt nachgewiesen zu werden, zu dem Genus Paramyxoviridae gehört.

8. Kit gemäß Anspruch 7, wobei das Virus das respiratorische Syncytialvirus ist.

9. Kit gemäß Anspruch 8, wobei das Virus, das in der Lage ist, durch eine Zielspezies direkt nachgewiesen zu werden, zu der Familie der Picorna Viren gehört.

10. Kit gemäß Anspruch 9, wobei das Picorna Virus, das in der Lage ist, durch eine Zielspezies direkt nachgewiesen zu werden, ein Maul- und Klauenseuche Virus ist.

11. Kit gemäß Anspruch 5, wobei die mikrobielle Zelle ausgewählt ist aus der Gruppe, bestehend aus einem Pilz, einer Hefe und einer bakteriellen Zelle.

12. Kit gemäß einem der Ansprüche 1 und 2, wobei die Zielspezies ausgewählt ist aus der Gruppe von Spezies, bestehend aus Antigenen; Haptenen; monoklonalen und polyklonalen Antikörpern; Gensonden; natürlichen und synthetischen Oligo- und Polynukleotiden; natürlichen und synthetischen Mono-, Oligo- und Polysacchariden; Lectinen; Avidin und Streptavidin; Biotin; Wachstumsfaktoren; Hormonen; Rezeptormolekülen; Protein A; und Protein G.

13. Kit gemäß einem der Ansprüche 1 und 2, wobei die Markierungsspezies ausgewählt ist aus der Gruppe von Spezies, bestehend aus Proteinen; Enzymen; Toxinen; Arzneimitteln; Farbstoffen; fluoreszierenden, lumineszierenden, phosphoreszierenden und anderen Licht emittierenden Substanzen; Metallchelatsubstanzen; Substanzen, die mit einem radioaktiven Isotop markiert sind; und Substanzen, die mit einem schweren Atom markiert sind.

14. Kit gemäß einem der Ansprüche 1 und 2, wobei die Markierungsspezies ausgewählt ist aus der Gruppe von Spezies, bestehend aus Ferritin, Phycoerythrinen, Phycocyaninen, Phycobilinen, Meerrettichperoxidase, alkalischer Phosphatase, Glucoseoxidasen, Galactosidasen, Ureasen, Iminodiessigsäure, Ethylendiamintetraessigsäure, Diethylentriaminpentaessigsäure und Desferrioxamin B.

15. Kit gemäß einem der Ansprüche 1 und 2, wobei die erste und zweite Zielspezies identisch sind.

16. Kit gemäß einem der Ansprüche 1 und 2, wobei die erste und zweite Zielspezies nicht identisch sind.

17. Kit gemäß einem der Ansprüche 1 und 2, wobei der polymere Träger ausgewählt ist aus der Gruppe von Polymeren, bestehend aus natürlichen und synthetischen Polysacchariden; Homopolyaminosäuren; natürlichen und synthetischen Polypeptiden und Proteinen; und synthetischen Polymeren mit nukleophilen funktionellen Gruppen.

18. Kit gemäß einem der Ansprüche 1 und 2, wobei der polymere Träger ausgewählt ist aus der Gruppe von Polymeren, bestehend aus Polyvinylalkoholen, Polyallylalkoholen, Polyethylenglykolen und substituierten Polyacrylaten.

19. Kit gemäß einem der Ansprüche 1 und 2, wobei der polymere Träger ausgewählt ist aus der Gruppe, bestehend aus Dextranen, Carboxymethyldextranen, Stärken, Hydroxyethylstärken, Hydroxypropylstärken, Glykogen, Agarosederivaten, Zellulosederivaten und natürlichen Gummis.

20. Kit gemäß Anspruch 7, wobei der polymere Träger ein Dextran ist.

21. Kit gemäß einem der Ansprüche 1 und 2, wobei der polymere Träger ausgewählt ist aus der Gruppe, bestehend aus Hydroxyethylzellulosen und Hydroxypropylzellulosen.

22. Kit gemäß einem der vorhergehenden Ansprüche, der Kit ist ein Tauchstäbchen.

23. Kit gemäß einem der vorhergehenden Ansprüche 1 - 22, der Kit ist für ein Mikrosystem angepasst.

24. Verfahren zum Nachweisen einer vorbestimmten biologischen Zelle, die in einer Probe anwesend ist, das Verfahren umfasst die Schritte
i) in Kontaktbringen der Probe mit dem Kit nach einem der Ansprüche 1 bis 19, und
ii) Nachweisen einer Zielspezies, die in der Lage ist, auf die vorbestimmte biologische Zelle abzuzielen,
wobei der Nachweis der Zielspezies die Anwesenheit der biologischen Zelle in der Probe angibt.

25. Verfahren gemäß Anspruch 24, wobei die Probe eine Körperflüssigkeitsprobe ist.

26. Verfahren gemäß Anspruch 24 oder 25 zum weiteren Nachweisen eines vorbestimmten Entzündungsindikators, der in einer Probe in einer Menge von weniger als ungefähr 2.000 pro Mikroliter (10⁻⁶ Liter) anwesend ist, das Verfahren umfasst die Schritte
i) in Kontaktbringen der Probe mit einem Kit, umfassend
a) einen festen Träger, und
b) eine Vielzahl einer ersten Zielspezies, die an den festen Träger gebunden ist, die Zielspezies ist in der Lage, den vorbestimmten Entzündungsindikator direkt zu binden, wenn er in einer Probe anwesend ist, die mit dem festen Träger in Kontakt gebracht wird, und
c) ein Konjugat, umfassend ein polymeres Trägermolekül, das an i) mindestens eine erste und/oder zweite Zielspezies, die in der Lage ist/sind, den vorbestimmten Entzündungsindikator direkt zu binden, wenn er in einer und ii) mindestens eine Markierungsspezies gebunden ist,
und
ii) Nachweisen einer Zielspezies, die in der Lage ist, auf den vorbestimmten Entzündungsindikator abzuzielen,
wobei der Nachweis der Zielspezies die Anwesenheit des vorbestimmten Entzündungsindikators in der Probe angibt.

27. Verfahren gemäß Anspruch 26, wobei das polymere Trägermolekül umfasst i) eine Vielzahl von mindestens einer reaktiven funktionellen Gruppe, ii) mindestens einen Verbindungsrest, der an die mindestens eine reaktive funktionelle Gruppe angeheftet ist, und iii) mindestens eine molekulare Spezies, ausgewählt aus der Gruppe molekularer Spezies, bestehend aus Zielspezies und Markierungsspezies, wobei jede der molekularen Spezies mindestens eine funktionelle Gruppe umfasst, die mit mindestens einem Verbindungsrest, der an das Reagens angeheftet ist, reaktiv ist, und wobei das Konjugat mindestens eine molekulare Spezies umfasst, die über einen Verbindungsrest daran angeheftet ist.

28. Verfahren gemäß einem der Ansprüche 24 - 27, wobei die Zielspezies ausgewählt ist aus der Gruppe von Spezies, bestehend aus Antigenen; Haptenen; monoklonalen und polyklonalen Antikörpern; Gensonden; natürlichen und synthetischen Oligo- und Polynukleotiden; natürlichen und synthetischen Mono-, Oligo- und Polysacchariden; Lectinen; Avidin und Streptavidin; Biotin; Wachstumsfaktoren; Hormonen; Rezeptormolekülen; Protein A; und Protein G.

29. Verfahren gemäß einem der Ansprüche 24 - 28, wobei die Markierungsspezies ausgewählt ist aus der Gruppe von Spezies, bestehend aus Proteinen; Enzymen; Toxinen; Arzneimitteln; Farbstoffen; fluoreszierenden, lumineszierenden, phosphoreszierenden und anderen Licht emittierenden Substanzen; Metallchelatsubstanzen; Substanzen, die mit einem radioaktiven Isotop markiert sind; und Substanzen, die mit einem schweren Atom markiert sind.

30. Verfahren gemäß einem der Ansprüche 24 - 29, wobei die Markierungsspezies ausgewählt ist aus der Gruppe von Spezies, bestehend aus Ferritin, Phycoerythrinen, Phycocyaninen, Phycobilinen, Meerrettichperoxidase, alkalischer Phosphatase, Glucoseoxidasen, Galactosidasen, Ureasen, Iminodiessigsäure, Ethylendiamintetraessigsäure, Diethylentriaminpentaessigsäure und Desferrioxamin B.

31. Verfahren gemäß der Ansprüche 24 - 30, wobei der polymere Träger ausgewählt ist aus der Gruppe von Polymeren, bestehend aus natürlichen und synthetischen Polysacchariden; Homopolyaminosäuren; natürlichen und synthetischen Polypeptiden und Proteinen; und synthetischen Polymeren mit nukleophilen funktionellen Gruppen.

32. Verfahren gemäß einem der Ansprüche 24 - 30, wobei der polymere Träger ausgewählt ist aus der Gruppe von Polymeren, bestehend aus Polyvinylalkoholen, Polyallylalkoholen, Polyethylenglykolen und substituierten Polyacrylaten.

33. Verfahren gemäß einem der Ansprüche 24 - 30, wobei der polymere Träger ausgewählt ist aus der Gruppe, bestehend aus Dextranen, Carboxymethyldextranen, Stärken, Hydroxyethylstärken, Hydroxypropylstärken, Glykogen, Agarosederivaten, Zellulosederivaten und natürlichen Gummis.

34. Verfahren gemäß Anspruch 32, wobei der polymere Träger ein Dextran ist.

35. Verfahren gemäß einem der Ansprüche 24 - 30, wobei der polymere Träger ausgewählt ist aus der Gruppe, bestehend aus Hydroxyethylzellulosen und Hydroxypropylzellulosen.

36. Verfahren gemäß einem der Ansprüche 26 bis 35, wobei der vorbestimmte Entzündungsindikator ausgewählt ist aus der Gruppe, bestehend aus Agonisten des IL-1 Systems, vorzugsweise IL-1α, IL-1β, IL-1ra, Autoantikörpern gegen IL-1α, sIL1-RI und sIL1-RII.

37. Verfahren gemäß einem der Ansprüche 26 bis 35, wobei der vorbestimmte Entzündungsindikator ausgewählt ist aus der Gruppe, bestehend aus Agonisten des TNFα Systems, vorzugsweise sTNFR p55 und p75.

38. Verfahren gemäß einem der Ansprüche 26 bis 35, wobei der vorbestimmte Entzündungsindikator ausgewählt ist aus der Gruppe, bestehend aus IL-6 und Autoantikörpern gegen IL-6.

39. Verfahren gemäß einem der Ansprüche 26 bis 35, wobei der vorbestimmte Entzündungsindikator ausgewählt ist aus der Gruppe, bestehend aus IL-12, sIL-4R, TNFβ (LT), INFγ, IL-4, und IL-10.

40. Verfahren gemäß einem der Ansprüche 26 bis 35, wobei der vorbestimmte Entzündungsindikator ausgewählt ist aus der Gruppe, bestehend aus IL-2, RANTES, IL-8, sIL-2R, IL-18, IFNα und eosinophilem kationischem Protein.

41. Verfahren zum Diagnostizieren eines infektiösen Zustandes in einem Individuum, das Verfahren umfasst die Schritte
i) Nachweisen einer vorbestimmten biologischen Zelle, die in einer Körperflüssigkeitsprobe gemäß einem der Ansprüche 24 - 40 anwesend ist, und
ii) Diagnostizieren des infektiösen Zustandes.

42. Verfahren gemäß Anspruch 41, das Verfahren umfasst die weiteren Schritte
i) Nachweisen eines vorbestimmten Entzündungsindikators, der in einer Körperflüssigkeitsprobe gemäß einem der Ansprüche 26 bis 40 anwesend ist, und
ii) Diagnostizieren des infektiösen Zustandes.

## Revendications

1. Trousse pour détecter directement une cellule biologique prédéterminée présente dans un échantillon en une quantité inférieure à environ 2000 par microlitre (10⁻⁶ litres) échantillons, ladite trousse comprenant
une phase solide étendue comprenant
i) un support solide, et
ii) une pluralité d'une première espèce de ciblage liée au support solide, ladite espèce de ciblage étant capable de se lier directement à ladite cellule biologique prédéterminée quand elle est présente dans un échantillon qui est amené en contact avec le support solide, et
une phase solide mobile comprenant
iii) un conjugué comprenant
une molécule support polymérique liée à
a) au moins une première et/ou une seconde espèces de ciblage capables de se lier directement à ladite cellule biologique prédéterminée quand elle est présente dans un échantillon qui est amené en contact avec le support solide, et
b) au moins une espèce de marquage, et
dans laquelle le conjugué comprend
A) une molécule support polymérique comprenant une pluralité d'au moins un groupe réactif fonctionnel,
B) au moins un fragment de connexion fixé audit au moins un groupe fonctionnel réactif,
C) au moins une espèce moléculaire choisie dans le groupe d'espèces moléculaires constitué par des espèces de ciblage et d'espèces de marquage, chacune des espèces moléculaires comprenant au moins un groupe fonctionnel qui est réactif avec au moins un fragment de connexion fixé au réactif,
D) dans laquelle le conjugué comprend au moins une espèce moléculaire fixée de manière covalente à celui-ci par un fragment de connexion,
dans laquelle ledit support solide est **caractérisé par** :
iv) une zone d'application pour appliquer l'échantillon comprenant une cellule biologique, ladite zone comprenant au moins un conjugué tel que défini en iii) ci-dessus, ledit conjugué étant mobile, et ladite zone d'application étant en contact liquide avec
v) une zone de détection pour détecter la présence, la quantité ou la concentration dudit au moins un conjugué, ladite zone comprenant en outre la pluralité d'une première espèce de ciblage liée au support solide.

2. Trousse selon la revendication 1, la trousse étant un dispositif de micro-écoulement ou un dispositif d'écoulement latéral.

3. Trousse selon l'une quelconque des revendications 1 à 3, dans laquelle la molécule support polymérique comprend des groupes fonctionnels réactifs en une quantité d'environ 5 à environ 5 000 µmol par gramme de support polymérique.

4. Trousse selon l'une quelconque des revendications 1 et 2, dans laquelle la cellule biologique qui peut être directement détectée par une espèce de ciblage est une cellule mammifère.

5. Trousse selon l'une quelconque des revendications 1 et 2, dans laquelle la cellule biologique qui peut être directement détectée par une espèce de ciblage est une cellule mammifère.

6. Trousse selon l'une quelconque des revendications 4 et 5, dans laquelle la cellule biologique qui peut être directement détectée par une espèce de ciblage est une particule virale

7. Trousse selon la revendication 6, dans laquelle le virus qui peut être directement détecté par une espèce de ciblage appartient au genre paramyxoviridae.

8. Trousse selon la revendication 7, dans laquelle le virus est un virus respiratoire syncytial.

9. Trousse selon la revendication 8, dans laquelle le virus qui peut être directement détecté par une espèce de ciblage appartient à la famille des picorna virus.

10. Trousse selon la revendication 9, dans laquelle le picorna virus qui peut être directement détecté par une espèce de ciblage est un virus de la fièvre aphteuse.

11. Trousse selon la revendication 5, dans laquelle la cellule microbienne est choisie dans le groupe constitué d'un champignon, d'une levure et d'une cellule bactérienne.

12. Trousse selon l'une quelconque des revendications 1 et 2, dans laquelle l'espèce de ciblage est choisie dans le groupe d'espèces constitué par des antigènes ; des haptènes ; des anticorps monoclonaux et polyclonaux ; des sondes géniques ; des oligo- et polynucléotides naturels et synthétiques ; des mono-, oligo- et polysaccharides naturels et synthétiques ; des lectines ; l'avidine et la streptavidine ; la biotine ; des facteurs de croissance ; des hormones ; des molécules réceptrices ; la protéine A ; et la protéine G.

13. Trousse selon l'une quelconque des revendications 1 et 2, dans laquelle l'espèce de marquage est choisie dans le groupe d'espèces constitué par des protéines ; des enzymes ; des toxines, des médicaments ; des colorants ; des substances fluorescentes, luminescentes, phosphorescentes et d'autres substances émettant de la lumière ; des substances chélatant un métal ; des substances marquées avec un isotope radioactif et de substances marquées avec un atome lourd.

14. Trousse selon l'une quelconque des revendications 1 et 2, dans laquelle l'espèce de marquage est choisie dans le groupe d'espèces constituées par la ferritine, des phycoérythrines, des phycocyanines, des phycobilines, la peroxydase de raifort, la phosphatase alcaline, des glucose oxydases, des galactosidases, des uréases, l'acide iminodiacétique, l'acide éthylènediaminotétraacétique, l'acide diéthylènetriaminopentaacétique et la desferrioxamine B.

15. Trousse selon l'une quelconque des revendications 1 et 2, dans laquelle la première et la seconde espèces de ciblage sont identiques.

16. Trousse selon l'une quelconque des revendications 1 et 2, dans laquelle la première et la seconde espèces de ciblage sont non-identiques.

17. Trousse selon l'une quelconque des revendications 1 et 2, dans laquelle le support polymérique est choisi dans le groupe de polymères constitué par des polysaccharides naturels et synthétiques ; des acides homopolyaminés ; des polypeptides et des protéines naturels et synthétiques et des polymères synthétiques ayant des groupes fonctionnels nucléophiles.

18. Trousse selon l'une quelconque des revendications 1 et 2, dans laquelle le support polymérique est choisi dans le groupe de polymères constitué par des poly(alcools vinyliques), des poly(alcools allyliques), des polyéthylèneglycols et des polyacrylates substitués.

19. Trousse selon l'une quelconque des revendications 1 et 2, dans laquelle le support polymérique est choisi dans le groupe constitué par des dextrans, des carboxyméthyl-dextrans, des amidons, des hydroxyéthylamidons, des hydroxypropylamidons, des glycogènes, des dérivés d'agarose, des dérivés de cellulose et des gommes naturelles.

20. Trousse selon la revendication 7, dans laquelle le support polymérique est un dextran.

21. Trousse selon l'une quelconque des revendications 1 et 2, dans laquelle le support polymérique est choisi dans le groupe constitué par des hydroxyéthylcelluloses et des hydroxypropylcelluloses.

22. Trousse selon l'une quelconque des revendications précédentes, ladite trousse étant sous la forme d'une bandelette réactive.

23. Trousse selon l'une quelconque des revendications 1 à 22 précédentes, ladite trousse étant adaptée pour un microsystème.

24. Procédé de détection d'une cellule biologique prédéterminée présente dans un échantillon, ledit procédé comprenant les étapes consistant à
i) mettre en contact l'échantillon avec la trousse selon l'une quelconque des revendications 1 à 19, et
ii) détecter une espèce de ciblage capable de cibler la cellule biologique prédéterminée,
dans lequel la détection de l'espèce de ciblage indique la présence de la cellule biologique dans l'échantillon.

25. Procédé selon la revendication 24, dans lequel l'échantillon est un échantillon de liquide organique.

26. Procédé selon la revendication 24 ou 25, pour détecter en outre un indicateur inflammatoire prédéterminé présent dans un échantillon en une quantité inférieure à environ 2000 par microlitre (10⁻⁶ litres), ledit procédé comprenant les étapes consistant à :
i) mettre en contact l'échantillon avec une trousse comprenant
a) un support solide, et
b) une pluralité d'une première espèce de ciblage liée au support solide, ladite espèce de ciblage étant capable de se lier directement audit indicateur inflammatoire prédéterminé quand il est présent dans un échantillon qui est amené en contact avec le support solide, et
c) un conjugué comprenant une molécule support polymérique liée à i) au moins une première et/ou une seconde espèces de ciblage capables de se lier directement audit indicateur inflammatoire prédéterminé quand il est présent dans un échantillon qui est amené en contact avec le support solide, et ii) au moins une espèce de marquage,
et
ii) détecter une espèce de ciblage capable de cibler l'indicateur inflammatoire prédéterminé,
dans lequel la détection de l'espèce de ciblage indique la présence de l'indicateur inflammatoire prédéterminé dans l'échantillon.

27. Procédé selon la revendication 26, dans lequel la molécule support polymérique comprend i) une pluralité d'au moins un groupe fonctionnel réactif, ii) au moins un fragment de connexion attaché à au moins un groupe fonctionnel réactif, et iii) au moins une espèce moléculaire choisie dans le groupe d'espèces moléculaires constitué par des espèces de ciblage et des espèces de marquage, dans lequel chacune des espèces moléculaire comprend au moins un groupe fonctionnel qui est réactif avec au moins un fragment de connexion fixé au réactif, et dans lequel le conjugué comprend au moins une espèce moléculaire fixée de manière covalente à celui-ci par un fragment de connexion.

28. Procédé selon l'une quelconque des revendications 24 à 27, dans lequel l'espèce de ciblage est choisie dans le groupe d'espèces constitué par des antigènes ; des haptènes ; des anticorps monoclonaux et polyclonaux ; des sondes géniques ; des oligo- et de polynucléotides naturels et synthétiques ; des mono-, oligo- et polysaccharides naturels et synthétiques ; des lectines ; l'avidine et la streptavidine ; la biotine ; des facteurs de croissance ; des hormones ; des molécules réceptrices ; la protéine A ; et la protéine G.

29. Procédé selon l'une quelconque des revendications 24 à 28, dans lequel l'espèce de marquage est choisie dans le groupe d'espèces constitué par des protéines ; des enzymes ; des toxines ; des médicaments ; des colorants ; des substances fluorescentes, luminescentes, phosphorescentes et d'autres substances émettant de la lumière ; des substances chélatant un métal ; des substances marquées avec un isotope radioactif et des substances marquées avec un atome lourd.

30. Procédé selon l'une quelconque des revendications 24 à 29, dans lequel l'espèce de marquage est choisie dans le groupe d'espèces constitué par la ferritine, des phycoérythrines, des phycocyanines, des phycobilines, la peroxydase de raifort, la phosphatase alcaline, des glucose oxydases, des galactosidases, des uréases, l'acide iminodiacétique, l'acide éthylènediaminotétraacétique, l'acide diéthylènetriaminopentaacétique et la desferrioxamine B.

31. Procédé selon l'une quelconque des revendications 24 à 30, dans lequel le support polymérique est choisi dans le groupe de polymères constitué par des polysaccharides naturels et synthétiques ; des acides homopolyaminés ; des polypeptides et des protéines naturels et synthétiques et des polymères synthétiques ayant des groupes fonctionnels nucléophiles.

32. Procédé selon l'une quelconque des revendications 24 à 30, dans lequel le support polymérique est choisi dans le groupe de polymères constitué par des poly(alcools vinyliques), des poly(alcools allyliques), des polyéthylèneglycols et des polyacrylates substitués.

33. Procédé selon l'une quelconque des revendications 24 à 30, dans lequel le support polymérique est choisi dans le groupe constitué par des dextrans, des carboxyméthyl-dextrans, des amidons, des hydroxyéthylamidons, des hydroxypropylamidons, des glycogènes, des dérivés d'agarose, des dérivés de cellulose et des gommes naturelles.

34. Procédé selon la revendication 32, dans lequel le support polymérique est un dextran.

35. Procédé selon l'une quelconque des revendications 24 à 30, dans lequel le support polymérique est choisi dans le groupe constitué par des hydroxyéthylcelluloses et des hydroxypropylcelluloses.

36. Procédé selon l'une quelconque des revendications 26 à 35, dans lequel l'indicateur inflammatoire prédéterminé est choisi dans le groupe constitué par des agonistes provenant du système d'IL-1, de préférence IL-1α, IL-1β, IL-1ra, des auto-anticorps contre IL-1α, sIL1-RI et sIL1-RII.

37. Procédé selon l'une quelconque des revendications 26 à 35, dans lequel l'indicateur inflammatoire prédéterminé est choisi dans le groupe constitué par des agonistes du système TNFα, de préférence sTNFR p55 et p75.

38. Procédé selon l'une quelconque des revendications 26 à 35, dans lequel l'indicateur inflammatoire prédéterminé est choisi dans le groupe constitué par IL-6 et des auto-anticorps contre IL-6.

39. Procédé selon l'une quelconque des revendications 26 à 35, dans lequel l'indicateur inflammatoire prédéterminé est choisi dans le groupe constitué par IL-12, sIL-4R, TNFβ (LT), INFγ, IL-4, et IL-10.

40. Procédé selon l'une quelconque des revendications 26 à 35, dans lequel l'indicateur inflammatoire prédéterminé est choisi dans le groupe constitué par IL-2, RANTES, IL-8, sIL-2R, IL-18, IFNα et une protéine cationique des éosinophiles.

41. Procédé pour le diagnostic d'un état infectieux chez un individu, ledit procédé comprenant les étapes consistant à :
i) détecter une cellule biologique prédéterminée présente dans un échantillon de liquide organique selon l'une quelconque des revendications 24 à 40, et
ii) diagnostiquer ledit état infectieux ;

42. Procédé selon la revendication 41, comprenant les étapes supplémentaires consistant à
i) détecter un indicateur inflammatoire prédéterminé présent dans un échantillon de liquide organique selon l'une quelconque des revendications 26 à 40, et
ii) diagnostiquer ledit état infectieux.
